# EUROPEAN PATENT SPECIFICATION

(11) **EP 1 830 824 B1**
(45) Date of publication and mention of the grant of the patent: **13.01.2016**
(21) Application number: 05821508.8
(22) Date of filing: 30.12.2005
(51) Int. Cl.: A61K 9/51, A61K 31/192, A61K 31/196, A61K 31/4985, A61K 31/5513, A61P 15/10, A61P 25/18, A61P 29/00

(54) **NANOPARTICLE COMPOSITION AND METHODS FOR SYNTHESIS THEREOF**
NANOTEILCHEN-ZUSAMMENSETZUNG UND VERFAHREN ZU IHRER SYNTHESE
COMPOSITION DE NANOPARTICULES ET PROCEDES DE SYNTHESE DE CETTE DERNIERE

(30) Priority: 31.12.2004 AU 2004907377
(43) Date of publication of application: 12.09.2007
(73) Proprietor: Iceutica Pty Ltd., Mouth Hawthorn, WA 6016 (AU)
(72) Inventor: MCCORMICK, Paul, Nedlands, W.A. 6907 (AU); DODD, Aaron, Nedlands Western Australia 6907 (AU); PAYNE, Trevor, Balingup Western Australia 6253 (AU); MEISER, Felix The University of Melbourne, Parkville, VIC 3052 (AU); POSTMA, Almar, Parkville, VIC 3052 (AU); CAMMARANO, Raffaele, Fremantle, W.A.6160 (AU); CARUSO, Frank The University of Melbourne, Parkville, VIC 3052 (AU); WILLIAMS, James, Kensington , W.A. 6151 (AU)
(74) Representative: D Young & Co LLP
(86) International application number: PCT/AU2005/001977
(87) International publication number: WO 2006/069419

(56) References cited:
- WO-A1-00/13672
- WO-A1-02/056866
- WO-A1-2005/032703
- WO-A2-02/094215
- WO-A2-2005/013937
- WO-A2-2005/020933
- WO-A2-2005/044234
- US-A- 5 145 684
- US-A- 5 298 262
- GRIGORIEVA T F ET AL: "Mechanosynthesis of nanocomposites", JOURNAL OF NANOPARTICLE RESEARCH, KLUWER ACADEMIC PUBLISHERS, DORDRECHT, NL, vol. 5, no. 5-6, 1 December 2003 (2003-12-01), pages 439-453, XP008121855, ISSN: 1388-0764
- GRIGORIEVA T.F., BARINOVA A.P., LYAKHOV N.Z.: 'Mechanosynthesis of nanocomposites' JOURNAL OF NANOPARTICLE RESEARCH vol. 5, no. 5-6, 2003, pages 439 - 453, XP008121855
- SCHAFFAZICK S.R. ET AL.: 'Freeze-drying polymeric colloidal suspensions: nanocapsules, nanospheres and nanodispersion. A comparative study' EUROPEAN JOURNAL OF PHARMACEUTICALS AND BIOPHARMACEUTICALS vol. 56, no. 3, November 2003, pages 501 - 505, XP004470489
- GUTERRES S.S. ET AL.: 'Poly(D,L-lactide) nanocapsules containing non-steroidal anti-inflammatory drugs: gastrointestinal tolerance following intravenous and oral administration' PHARMACEUTICAL RESEARCH vol. 12, no. 10, October 1995, pages 1545 - 1547, XP008121856

## Description

### Field of the Invention

The present invention relates to improved therapeutically active nanocomposite microstructure compositions, including nanoparticle compositions comprising nanoparticles of a therapeutically active agent dispersed in a carrier matrix and other nanoparticle preparations. The invention also relates to a method for preparing said compositions and preparations using solid-state mechanochemical synthesis. Further, it relates to therapeutic products produced using said compositions and to methods of treatment using the compositions.

### Background

Poor bioavailability is a significant problem encountered in the development of therapeutic compositions, particularly those compounds containing an active agent that is poorly soluble in water. An active agent's bioavailability is the degree to which the active agent becomes available to the target tissue in the body after systemic administration through, for example, oral or intravenous means. Many factors may affect bioavailability, including the form of dosage and the solubility and dissolution rate of the active agent.

Poorly and slowly water soluble active agents tend to be eliminated from the gastrointestinal tract before being absorbed into the circulation. In addition, poorly soluble active agents tend to be disfavored or even unsafe for intravenous administration due to the risk of particles of agent blocking blood flow through capillaries.

It is known that the rate of dissolution of a particulate drug can increase with increasing surface area, that is, decreasing particle size. Consequently, methods of making finely divided or sized drugs have been studied and efforts have been made to control the size and size range of drug particles in pharmaceutical compositions. For example, dry milling techniques have been used to reduce particle size and hence influence drug absorption. However, in conventional dry milling the limit of fineness is reached generally in the region of about 100 microns (100,000 nm), at which point material cakes on the milling chamber and prevents any further diminution of particle size. Alternatively, wet grinding may be employed to reduce particle size, but flocculation restricts the lower particle size limit to approximately 10 microns (10,000 nm). The wet milling process, however, is prone to contamination, thereby leading to a bias in the pharmaceutical art against wet milling. Another alternative milling technique, commercial airjet milling, has provided particles ranging in average size from as low as about 1 to about 50 microns (1,000-50,000 nm).

There are several approaches currently used to formulate poorly soluble active agents. One approach is to prepare the active agent as a soluble salt. Where this approach cannot be employed, alternate (usually physical) approaches are employed to improve the solubility of the active agent. Alternate approaches generally subject the active agent to physical conditions which change the agent's physical and or chemical properties to improve its solubility. These include process technologies such as micro-ionisation, modification of crystal or polymorphic structure, development of oil based solutions, use of co-solvents, surface stabilizers or complexing agents, micro-emulsions, super critical fluid and production of solid dispersions or solutions. More than one of these processes may be used in combination to improve formulation of a particular therapeutic compound.

These techniques for preparing such pharmaceutical compositions tend to be complex. By way of example, a principal technical difficulty encountered with emulsion polymerization is the removal of contaminants, such as unreacted monomers or initiators (which may have undesirable levels of toxicity), at the end of the manufacturing process.

Another method of providing reduced particle size is the formation of pharmaceutical drug microencapsules, which techniques include micronizing, polymerisation and co-dispersion. However, these techniques suffer from a number of disadvantages including at least the inability to produce sufficiently small particles such as those obtained by milling, and the presence of co-solvents and/or contaminants such as toxic monomers which are difficult to remove, leading to expensive manufacturing processes.

Over the last decade intense scientific investigation has been carried out to improving the solubility of active agents by converting the agents to ultra fine powders by methods such as milling and grinding. These techniques may be used to increase the dissolution rate of a particulate solid by increasing the overall surface area and decreasing the average particle size.

Some investigation of the applicability of mechanochemical synthesis ("MCS") techniques to active agents has been undertaken. However, these investigations have focused on providing an alternative manufacturing process that reduces the need for solvents and improves yields, rather than improving solubility by reducing particle size.

It is important to note the clear distinction between the MCS method, described more fully below in the Detailed Description of the Invention, which is one of building nanoparticles from chemical precursors, as compared to a particle size reduction methods.

Methods of making nanoparticulate compositions have been described as early as US Pat. No. 5,145,684. Methods of making nanoparticulate compositions are also described in U.S. Pat. Nos. 5,534,270; 5,510,118; 5,470,583; 5,591,456; 6,428,814; 6,811,767; and 6,908,626, all of which are specifically incorporated herein by reference. However, these patents do not teach MCS methods of forming nanoparticulate compositions. Rather, the techniques described therein are size reduction techniques. Additionally, these techniques do not result in nanoparticulate compositions with average particle sizes in the range of the present invention's particles, nor do they teach the matrix carrier feature of some embodiments of the present invention.

Accordingly the present invention seeks to provide improved therapeutically active nanocomposite microstructure compositions and nanoparticle preparations as well as methods for their preparation, which at least ameliorate some of the problems attendant with prior technologies.

### Summary of the Invention

The present invention is directed to the surprising and unexpected discovery that improved nanocomposite microstructure compositions can be produced by mechanochemically synthesising therapeutically active nanoparticles in a carrier matrix using a solid-state chemical reaction. By mechanochemically synthesising the therapeutically active nanoparticles in a carrier matrix using mechanochemical procedures, applicant is able to control the size of the resultant nano particles in the composition. As a result, the improved nanocomposite microstructure compositions are expected to have several advantages, including improved drug bioavailability compared to unprocessed or conventional active agents.

Accordingly, the present invention relates to an improved nanocomposite microstructure composition comprising therapeutically active nanoparticles dispersed in a carrier matrix, wherein said composition is mechanochemically prepared using a solid-state chemical reaction. Preferably, the preparation is a solid solution or solid dispersion suitable for delivery to an animal.

The present invention also resides in a method for preparing an improved nanocomposite microstructure composition, said method comprising the step of: contacting a precursor compound with a co-reactant under mechanochemical synthesis conditions to generate a solid-state chemical reaction between the precursor compound and the co-reactant to produce therapeutically active nanoparticles dispersed in a carrier matrix. The carrier matrix produced by this method will preferably be non-toxic or alternatively should be separable from the therapeutically active nanoparticles.

The present invention also relates to the use of the composition of the invention in the manufacture of a medicament. Such a medicament may include the composition alone or more preferably the composition may be combined with one or more pharmaceutically acceptable carriers, as well as any desired excipients or other like agents commonly used in the preparation of pharmaceutically acceptable compositions.

The present invention is further directed to methods of treatment of an animal comprising administering to said animal a therapeutically effective amount of a composition produced according to a method of the invention, wherein said animal is in need of said therapeutically active agent.

One aspect of the invention relates to a method for preparing a purified nanoparticulate therapeutically active agent comprising the step of:
(i) contacting a precursor compound with a co-reactant under mechanochemical synthesis conditions wherein a solid-state chemical reaction between the precursor compound and the co-reactant produces therapeutically active nanoparticles dispersed in a carrier matrix.

In another aspect of the invention, the method further comprises the step of:
(ii) removing a desired amount of the carrier matrix to release the therapeutically active nanoparticles.

The step of removing a desired amount of the carrier matrix to release the therapeutically active nanoparticles may be performed through means such as selective dissolution, washing, or sublimation.

The invention also extends to the product of the aforementioned methods and its use in the preparation of medicaments and therapeutically active compositions suitable for treating an animal, such as a human. The invention includes methods for preparing medicaments and pharmaceutically acceptable compositions comprising the purified nano-particulate therapeutically active agent.

Thus, in one aspect, the invention includes a method of producing a nanoparticle composition comprising nanoparticles of a therapeutically effective agent, comprising the step of: mechanochemical synthesis of a mixture of a precursor compound and a co-reactant using milling media in a milling apparatus, for a time period sufficient to produce the nanoparticle composition comprising nanoparticles of the therapeutically effective agent dispersed within a carrier matrix. The nanoparticles may have an average size less than 200 nm, 100 nm, 75 nm, 50 nm, or 40 nm. Further, the size distribution of the nanoparticles may be such that at least 50% of the nanoparticles, or 75% of the nanoparticles, is within the specified average size range. The time period varies, depending on the nature of the reactants, and may range from between 5 minutes and 2 hours, 5 minutes and 1 hour, 5 minutes and 45 minutes, 5 minutes and 30 minutes, and 10 minutes and 20 minutes. The milling media may have a diameter between 1 and 20mm, or between 2 and 15 mm, or between 3 and 10mm.

In another aspect of the invention, the precursor compound may be selected from biologics, amino acids, proteins, peptides, nucleotides, nucleic acids, and analogs thereof. Further, the precursor compound may be selected from a variety of classes of drugs, including anti-obesity drugs, central nervous system stimulants, carotenoids, corticosteroids, elastase inhibitors, anti-fungals, oncology therapies, anti-emetics, analgesics, cardiovascular agents, anti-inflammatory agents, such as NSAIDs and COX-2 inhibitors, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives (hypnotics and neuroleptics), astringents, alpha-adrenergic receptor blocking agents, beta-adrenoceptor blocking agents, blood products and substitutes, cardiac inotropic agents, contrast media, corticosteroids, cough suppressants (expectorants and mucolytics), diagnostic agents, diagnostic imaging agents, diuretics, dopaminergics (antiparkinsonian agents), haemostatics, immunological agents, lipid regulating agents, muscle relaxants, parasympathomimetics, parathyroid calcitonin and biphosphonates, prostaglandins, radio-pharmaceuticals, sex hormones (including steroids), anti-allergic agents, stimulants and anoretics, sympathomimetics, thyroid agents, vasodilators, and xanthines. In other aspects, the precursor compound may be selected from haloperidol, DL isoproterenol hydrochloride, terfenadine, propranolol hydrochloride, desipramine hydrochloride, salmeterol, sildenafil citrate, tadalafil, vardenafil, fenamic acids, Piroxicam, Naproxen, Voltaren (diclofenac), rofecoxib, ibuprofren ondanstetron, sumatriptan, naratryptan, ergotamine tartrate plus caffeine, methylsegide, olanzapine.

In another aspect of the invention, the method may comprise an additional step of removing at least a portion of the carrier matrix, wherein the nanoparticles remaining have an average particle size of less than 200 nm. Any portion of the carrier matrix may be removed, including but not limited to 25%, 50%, 75%, or substantially all of the carrier matrix removed.

In another aspect, the invention is directed to nanoparticle compositions produced by any of the foregoing methods. The invention is also directed to pharmaceutical compositions having at least the nanoparticle compositions and a pharmaceutically acceptable carrier. Medicaments may also be manufactured in accordance with the methods of the invention, combining a therapeutically effective amount of a nanoparticle composition produced thereby with a pharmaceutically acceptable carrier.

In another aspect, the invention is directed to a nanoparticle composition comprising nanoparticles of a therapeutically effective agent dispersed in a carrier matrix, which nanoparticles have an average size selected from less than 200 nm, less than 100 nm, less than 75 nm, less than 50 nm, and less than 40 nm. The nanoparticle size distribution may be such that at least 50% of the nanoparticles, or 75% of the nanoparticles, is within the specified average size range.

In one aspect the invention includes a nanoparticle composition wherein the carrier matrix is selected from Na₂CO₃, NaHCO₃, NH₄Cl, and NaCl, or an appropriate combination thereof. In another aspect of the invention, the precursor compound is selected from diclofenac, naproxen, olanzapine, and sildenafil.

In another aspect the invention is directed to a nanoparticle composition comprising nanoparticles of a therapeutically effective agent dispersed in a carrier matrix, the nanoparticle composition being formed by a process comprising the step of mechanochemical synthesis of a mixture of a precursor compound and a co-reactant using milling media in a milling apparatus, for a time period sufficient to produce the nanoparticle composition. A nanoparticle composition of the invention may also be produced by a process having an additional step of removing at least a portion of the carrier matrix. The foregoing options of nanoparticle size, MCS time, precursor compound, and carrier matrix are applicable to these nanoparticle compositions as well.

Other aspects and advantages of the invention will become apparent to those skilled in the art from a review of the ensuing description.

### Brief Description of the Drawings

Figure 1: XRD traces of (a) precipitated diclofenac acid (upper line) and (b) a stoichiometric mixture of diclofenac sodium and sodium hydrogen sulfate, milled for 6 hours (lower line).
Figure 2: TG-DSC traces of precipitated diclofenac acid (upper line); and diclofenac-sodium (2.90g) plus sodium hydrogen sulfate (1.18g), milled for 6 hours (lower line).
Figure 3: Transmission electron microscope image of nanoparticle composition formed by MCS comprising diclofenac sodium salt dispersed in a matrix carrier comprising sodium carbonate and sodium bicarbonate.
Figure 4: Transmission electron microscope image of nanoparticle composition formed by MCS comprising diclofenac acid dispersed in a matrix carrier comprising ammonium chloride.
Figure 5: Transmission electron microscope image of nanoparticle composition formed by MCS comprising naproxen acid dispersed in a matrix carrier comprising ammonium chloride.

### Detailed Description of the Invention

### General

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in the specification, individually or collectively and any and all combinations or any two or more of the steps or features.

The present invention is not to be limited in scope by the specific embodiments described herein, which are intended for the purpose of exemplification only. Functionally equivalent products, compositions and methods are clearly within the scope of the invention as described herein.

The invention described herein may include one or more ranges of values (e.g. size, concentration etc). A range of values will be understood to include all values within the range, including the values defining the range, and values adjacent to the range which lead to the same or substantially the same outcome as the values immediately adjacent to that value which defines the boundary to the range.

The entire disclosures of all publications (including patents, patent applications, journal articles, laboratory manuals, books, or other documents) cited herein are hereby incorporated by reference. No admission is made that any of the references constitute prior art or are part of the common general knowledge of those working in the field to which this invention relates.

As used herein the term "derived" and "derived from" shall be taken to indicate that a specific integer may be obtained from a particular source albeit not necessarily directly from that source.

Throughout this specification, unless the context requires otherwise, the word "comprise", or variations such as "comprises" or "comprising", will be understood to imply the inclusion of a stated integer or group of integers but not the exclusion of any other integer or group of integers.

Throughout this specification, unless the context requires otherwise, the word "comprise" or variations, such as "comprises" or "comprising" will be understood to imply the inclusion of a stated integer, or group of integers, but not the exclusion of any other integers or group of integers. It is also noted that in this disclosure, and particularly in the claims and/or paragraphs, terms such as "comprises", "comprised", "comprising" and the like can have the meaning attributed to it in US Patent law; e.g., they can mean "includes", "included", "including", and the like; and that terms such as "consisting essentially of" and "consists essentially of" have the meaning ascribed to them in US patent law, for example they allow for elements not explicitly recited, but exclude elements that are not in the prior art or that affect a basic or novel characteristic of the invention.

As used herein the term "nanocomposite microstructure" includes nanoparticle compositions, wherein the composition comprises at least nanoparticles having an average particle size smaller than 1000 nm. Nanocomposite microstructure as used herein also includes "nanoparticulate therapeutically active agent" and the like. Drug nanoparticles dispersed in a carrier matrix are included in nanocomposite microstructures, as are embodiments thereof wherein the carrier matrix has been partially or substantially wholly removed.

"Conventional active agents or drugs" refers to non-nanoparticulate compositions of active agents or solubilized active agents or drugs. Non-nanoparticulate active agents have an effective average particle size of greater than about 2 microns, meaning that at least 50% of the active agent particles have a size greater than about 2 microns. (Nanoparticulate active agents as defined herein have an effective average particle size of less than about 1000 nm.)

"Therapeutically effective amount" as used herein with respect to a drug dosage, shall mean that dosage that provides the specific pharmacological response for which the drug is administered in a significant number of subjects in need of such treatment. It is emphasized that "therapeutically effective amount," administered to a particular subject in a particular instance will not always be effective in treating the diseases described herein, even though such dosage is deemed a "therapeutically effective amount" by those skilled in the art. It is to be further understood that drug dosages are, in particular instances, measured as oral dosages, or with reference to drug levels as measured in blood.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

### Mechanochemical Synthesis

The term "mechanochemical synthesis" ("MCS") means the use of mechanical energy to activate, initiate or promote a chemical reaction, a crystal structure transformation or a phase change in a material or a mixture of materials, for example by agitating a reaction mixture in the presence of a milling media to transfer mechanical energy to the reaction mixture, and includes without limitation "mechanochemical activation", "mechanochemical processing", "reactive milling", and related processes. The reaction mixture can be contained in a closed vessel or chamber. The term "agitating" or "agitation" as used herein means applying at least one, or any combination of two or more of the fundamental kinematic motions including translation (e.g., side-to-side shaking), rotation (e.g., spinning or rotating) and inversion (e.g., end-over-end tumbling) to the reaction mixture. Preferably, all three motions are applied to the reaction mixture. Such agitation can be accomplished with or without external stirring of the reaction mixture and milling media.

In the MCS process of the present invention, a mixture of reactants, in the form of crystals, powders, or the like, is combined in suitable proportions with milling media in a vessel or chamber that is mechanically agitated (i.e., with or without stirring) for a predetermined period of time at a predetermined intensity of agitation. Typically, a milling apparatus is used to impart motion to the milling media by the external application of agitation, whereby various translational, rotational or inversion motions or combinations thereof are applied to the vessel or chamber and its contents, or by the internal application of agitation through a rotating shaft terminating in a blade, propeller, impeller or paddle or by a combination of both actions. Processes that can be mechanically activated by the methods described herein may include: initiation of chemical reactions, for example, solid state reactions such as, oxidation/reduction reactions, ionexchange reactions, substitution reactions, etc.; dehydration; generation of dislocations in crystal lattices; initiation of polymorphic phase transformations; formation of metastable phases; refinement of crystallite size; amorphization of crystalline phases; formation of salts from free acids or bases, and free acids or bases from salts and the like.

Such processes can be promoted under nominally ambient conditions in the absence of added liquids or solvents.

A detailed description of various aspects of mechanochemical processing is provided by P. G. McCormick and F. H. Froes ("The Fundamentals of Mechanochemical Processing", Journal of Metals, vol. 50, 1998, pp 61-65) and E. M. Gutman ("Mechanochemistry of Materials", Cambridge Internat. Science Publ., 1998) and references cited therein.

In the method of the present invention, a predetermined amount of milling media, preferably chemically-inert, rigid milling media, is added to an essentially dry reaction mixture comprising at least a precursor composition (ordinarily a form of pharmaceutical drug) and a co-reactant, prior to mechanical activation. The reaction mixture is subjected to mechanical activation, for example, in a milling apparatus whereby the reaction mixture is agitated in the presence of milling media at ambient temperature, that is, without the need for external heating. The term "chemically-inert" milling media, as used herein, means that the milling media does not react chemically with any of the components of the reaction mixture.

Typically, rigid milling media can be in the form of particles desirably having a variety of smooth, regular shapes, flat or curved surfaces, and lacking sharp or raised edges. For example, suitable milling media can be in the form of particles having ellipsoidal, ovoid, spherical or right cylindrical shapes. Preferably, the milling media is in the form of beads, balls, spheres, rods, right cylinders, drums or radius-end right cylinders (i.e., right cylinders having hemispherical bases with the same radius as the cylinder). Depending on the nature of the precursor compound and the co-reactant, the milling media desirably has an effective mean particle diameter (i.e., "particle size") between about 0.1 and 30 mm, more preferably between about 1 and about 15 mm, still more preferably between about 3 and 10 mm. As used herein, the term "effective mean particle diameter" is defined as the mean diameter of the smallest circular hole through which a particle can pass freely. For example, the effective mean particle diameter of a spherical particle corresponds to the mean particle diameter and the effective mean particle diameter of an ellipsoidal particle corresponds to the mean length of the longest minor axis.

The rigid milling media advantageously comprises various materials such as ceramic, glass, metal or polymeric compositions, in a particulate form. Suitable metal milling media are typically spherical and generally have good hardness (i.e., RHC 60-70), roundness, high wear resistance, and narrow size distribution and can include, for example, balls fabricated from type 52100 chrome steel, type 316 or 440C stainless steel or type 1065 high carbon steel.

Preferred ceramic materials, for example, can be selected from a wide array of ceramics desirably having sufficient hardness and resistance to fracture to enable them to avoid being chipped or crushed during milling and also having sufficiently high density. Suitable densities for milling media can range from about 1 to 15 g/cm³. Preferred ceramic materials can be selected from steatite, aluminum oxide, zirconium oxide, zirconia-silica, yttria-stabilized zirconium oxide, magnesia-stabilized zirconium oxide, silicon nitride, silicon carbide, cobalt-stabilized tungsten carbide, and the like, as well as mixtures thereof..

Preferred glass milling media are spherical (e.g., beads), have a narrow size distribution, are durable, and include, for example, lead-free soda lime glass and borosilicate glass. Polymeric milling media are preferably substantially spherical and can be selected from a wide array of polymeric resins having sufficient hardness and friability to enable them to avoid being chipped or crushed during milling, abrasion-resistance to minimize attrition resulting in contamination of the product, and freedom from impurities such as metals, solvents, and residual monomers.

Preferred polymeric resins, for example, can be selected from crosslinked polystyrenes, such as polystyrene crosslinked with divinylbenzene, styrene copolymers, polyacrylates such as polymethylmethacrylate, polycarbonates, polyacetals, vinyl chloride polymers and copolymers, polyurethanes, polyamides, high density polyethylenes, polypropylenes, and the like. The use of polymeric milling media to grind materials down to a very small particle size (as opposed to mechanochemical synthesis) is disclosed, for example, in U.S. Pat. Nos. 5,478,705 and 5,500,331. Polymeric resins typically can have densities ranging from about 0.8 to 3.0 g/cm³. Higher density polymeric resins are preferred. Alternatively, the milling media can be composite particles comprising dense core particles having a polymeric resin adhered thereon. Core particles can be selected from materials known to be useful as milling media, for example, glass, alumina, zirconia silica, zirconium oxide, stainless steel, and the like. Preferred core materials have densities greater than about 2.5 g/cm³.

In one form of the invention, the milling media are formed from a ferromagnetic material, thereby facilitating removal of contaminants arising from wear of the milling media by the use of magnetic separation techniques.

During mechanical activation, the milling media facilitate the direct transfer of mechanical energy generated by the interaction of the milling media and the milling apparatus during the rotation, translation, inversion or agitation thereof to the reactant powders without significant bulk heating of the mixture. Motion imparted to the milling media can result in application of shearing forces as well as multiple impacts or collisions having significant intensity between milling media particles and particles of the reactant powders. The efficiency of mechanical energy transfer from the milling media to the reactant particles is influenced by a wide variety of processing parameters including: the type of milling apparatus; the intensity of the forces generated, the kinematic aspects of the process; the size, density, shape, and composition of the milling media; the weight ratio of the reaction mixture to the milling media; the duration of activation; the physical properties of both reactants and products; the atmosphere present during activation; and also others. The general physical and chemical principles governing mechanochemical activation are incompletely understood and ordinarily depend on the specific reactants and products.

The mechanical activation process of the present invention is accomplished most advantageously by a milling apparatus that can repeatedly or continuously apply mechanical compressive forces and shear stress to the particles of the reaction mixture. A suitable milling apparatus can be any reactor or vessel that facilitates energy exchange between the precursor compound and the co-reactant and may be selected from any known in the art, including but not limited to the following: high-energy ball, sand, bead or pearl mills, basket mill, planetary mill, vibratory action ball mill, multi-axial shaker/mixer, stirred ball mill, horizontal small media mill, multi-ring pulverizing mill, and the like, including small milling media. The milling apparatus also can contain one or more rotating shafts. Suitable rates of agitation and total activation times are adjusted for the type and size of milling apparatus as well as the milling media, the weight ratio of the reaction mixture to milling media, the chemical and physical properties of the reactants and products, and other parameters that may be optimized empirically.

A solid state mechanochemical reaction is one that induces chemical processes in molecular solids by mechanical treatment of the sample. Mechanical milling or grinding is the usual way to achieve such mechanical activation of the solid. More preferably, it is achieved by mechanical milling in a ball mill. Throughout the remainder of the specification reference will be made to mechanical activation being carried out inside a ball mill. Examples of this type of mill are attritor mills, nutating mills, tower mills, planetary mills, vibratory mills and gravity-dependent-type ball mills. It will be appreciated that the mechanical activation may also be achieved by any suitable means other than ball milling. For example, mechanical activation may also be achieved using jet mills, rod mills, roller mills or crusher mills.

Mechanical activation occurs in a ball mill when grinding media, typically steel or ceramic balls, are kept in a state of continuous relative motion with a feed material by the application of mechanical energy, such that the energy imparted to the feed material during ball-feed-ball and ball-feed-liner collisions is sufficient to cause mechanical activation of the solid.

The particle size formed during the milling step is a function of the milling process employed, the size of the milling balls and the period of time for milling. Of particular importance is the collision energy associated with ball to powder collisions. Important determinants of collision energy include ball to powder mass ratio, and ball size. If the collision energy is too high during milling, the chemical reaction may be unstable, resulting in melting and vaporisation of the product phase and a significant increase in particle size and changes in product morphology. The collision energy required to cause combustion is a function of the enthalpy change associated with the reaction as well as other factors.

Preferably, the reactants are milled for the shortest time necessary to form the nanocomposite microstructure composition C+D as described below, to minimise any possible contamination from the mill and milling media. This time varies greatly, depending on the reactants, and may range from as short as 5 minutes to several hours. However, unlike the MCS processes known in the art for inorganic compounds, the inventors have found surprisingly that MCS milling times of about 15 minutes are usually sufficient to successfully achieve nanoparticle formation. MCS milling times in excess of 2 hours may lead to degradation of the nanoparticle formulation and an increased level of undesirable contaminants. In some cases, it may be necessary to undertake a subsequent heat-treatment of the products to ensure the reaction has progressed to completion.

To facilitate the formation of separated, discrete nanoparticles within the carrier matrix, regard must be had to the volume fraction of these nanoparticles. For example, it may be desirable for the volume fraction of the nanoparticles to be less than the theoretical percolation threshold, which, for 3-dimensional random dispersions of spherical particles, is around 15%.

### Nanocomposite microstructures (nanoparticle compositions)

The present invention provides an improved nanocomposite microstructure composition comprising therapeutically active nanoparticles, wherein said preparation is mechanochemically prepared using a solid-state chemical reaction as described above. In one form, the present invention provides an improved nanocomposite microstructure composition comprising therapeutically active nanoparticles dispersed in a carrier matrix, wherein said preparation is mechanochemically prepared using a solid-state chemical reaction as described above. By generating the nanocomposite microstructure composition using solid-state chemistry in a mechanochemical reaction, applicant is able to control the concentration and distribution of the therapeutically active nanoparticles within the carrier matrix as well as the size and potentially the polymorphic structure of the nanoparticles.

Nanocomposite microstructure compositions of the invention have potentially beneficial pharmacokinetic properties as compared with alternately produced material. A smaller particle size results in enhanced dissolution rate at least in part as a result of the increased surface area of the composition. The production method also has the potential to induce different polymorphic structures in the compounds which also have the potential to favourably affect solubility. Altering particle size or polymorphic structure has been documented to improve bioavailability, improve dose proportionality, reduce fed/fasted variability, reduce inter-subject variability, and enhance absorption rate.

Where the carrier matrix is selectively substantially removed to leave pure therapeutically active nanoparticles, agglomeration of the particles may sometimes occur forming larger particles. Due to the unique nature of the process described, these new agglomerated particles may have unique physical properties, through, for instance, having new polymorphic structures or nano-structured processes. As described previously, unique polymorphic structures and or the presence of nano structures processes may result in therapeutically beneficial properties including improved bioavailability. Thus, in some embodiments of the invention, a composition of the invention comprises substantially pure therapeutically active nanoparticles. In other embodiments, particularly where the lack of carrier matrix allows the nanoparticles formed during the process to agglomerate in a way detrimental to improving the dissolution rate, the preferred composition retains at least a portion of the carrier matrix.

The nanoparticles within the microstructure composition are present at a concentration of between about 0.1% and about 99.0% by weight. Preferably, the concentration of therapeutically active nanoparticles within the microstructure composition will be about 5% to about 80% by weight, while concentrations of 10% to about 50% by weight are highly preferred. Desirably, the concentration will be in the range of about 10 to 15% by weight, 15 to 20% by weight, 20 to 25% by weight, 25 to 30% by weight, 30 to 35% by weight, 35 to 40% by weight, 40 to 45% by weight or 45 to 50% by weight for the unwashed microstructure composition prior to any later removal (if desired) of any portion of the carrier matrix. Where part or all of the matrix has been removed the relative concentration of nanoparticles in the composition may be considerably higher depending on the amount of the matrix that is removed. For example, if all of the matrix is removed the concentration of nanoparticles in the preparation may be approximately 100% by weight.

The dispersion of nanoparticles in the carrier matrix will be dependent on the weight percentage concentration of active agent in the microstructure. Depending on that weight percentage concentration, nanoparticles in the microstructure will be "dispersed" if at least 0.1% of the nanoparticles are separated by the carrier matrix. Preferably, greater than 10% of the nanoparticles in the microstructure will be spatially separated from each other by the carrier matrix. More preferably at least 15, 20, 30, 40, 50, 55, 60, 65, 70, 75, 80, 85, 90, 92, 95, 98 or 99% of the nanoparticles in the microstructure will be spatially separated from each other by the carrier matrix.

Therapeutically active nanoparticles within the microstructure composition will preferably be in a size range of about 1 nm to about 200 nm, or more preferably in the range of about 5 nm to about 100 nm, more preferably between about 5 and 50 nm, more preferably still in the range of about 10 nm to about 40 nm. In a highly preferred embodiment of the invention, the nanoparticles will be between about 20 nm to 30 nm in size. These sizes refer to nanoparticles either fully dispersed or partially agglomerated. For example, where two 20 nm particles agglomerate, the resulting entity is a nanoparticle about 40 nm in size and thus would still be considered a nanoparticle of the invention. Stated alternatively, the therapeutically active nanoparticles will preferably have an average size less than 200 nm, more preferably less than 100 nm, more preferably less than 75 nm, more preferably less than 50 nm, and more preferably less than 40 nm, where the average size refers to nanoparticles either fully dispersed or partially agglomerated as described above.

Nanocomposite microstructure compositions produced according to the present invention are not limited to the inclusion of a single species of therapeutically active nanoparticle in the carrier matrix. More than one species of therapeutically active nanoparticle may therefore be present in the composition. Where more than one species of nanoparticle is present, the nanocomposite microstructure composition so formed may either be prepared in a single solid-state mechanochemical reaction or more preferably the reactions may be allowed to occur separately and the reaction products are then brought together to form a single nanocomposite microstructure composition.

In another embodiment, a nanoparticle composition of the invention may be combined into a therapeutic with another therapeutically active agent, or even the same agent. In the latter embodiment, a therapeutic dosage form may be achieved which provides for different release characteristics - early release from the nanoparticulate agent, and later release from a larger average size nanoparticulate agent or a non-nanoparticulate agent.

In one embodiment of the invention, the nanocomposite microstructure composition is preferably a solid solution or solid dispersion. A solid solution consists of one phase only, irrespective of the number of differing components present. A solid solution may be classified as continuous, discontinuous, substitutional, interstitial or amorphous. Typical solid solutions have a crystalline structure, in which the solute molecules can either substitute for solvent molecules in the crystal lattice or fit into the interstices between the solvent molecules. Interstitial crystalline solid solutions occur when the dissolved molecules occupy the interstitial spaces between the solvent molecules in the crystal lattice. Amorphous solid solutions occur when the solute molecules are dispersed molecularly but irregularly within the amorphous solvent.

According to the invention, preferably the solid solution or dispersion comprises the therapeutically active nanoparticle distributed within a carrier matrix. More preferably, the solid solution or dispersion will comprise stabilizers that are therapeutically inert and/or non-toxic. If the therapeutic nanoparticles can be formulated into a solid dosage form ( eg, for oral or suppository administration), while being retained in the carrier matrix, then there will be little or no need for further stabilizing the dispersion since the carrier matrix effectively acts as a solid-state stabilizer.

However, if the nanoparticles are to be utilized in a liquid (or gaseous) suspension, the nanoparticles may require further stabilization once the solid carrier has been substantially removed to ensure the elimination, or at least minimisation of particle agglomeration.

### Method for preparing improved nanocomposite microstructure compositions

The present invention includes a method for preparing an improved nanocomposite microstructure compositions, said method comprising the step of: contacting a precursor compound with a co-reactant under mechanochemical synthesis conditions to generate a solid-state chemical reaction between the precursor compound and the co-reactant, yielding therapeutically active nanoparticles dispersed in a carrier matrix. The carrier matrix produced by this method will preferably be non-toxic or alternatively should be separable and/or substantially removable from the therapeutically active nanoparticles.

Preferably, the improved nanocomposite microstructure composition is formed by the following general reaction:

A + B → C + D

where A is the precursor compound; B is the co-reactant; C is the carrier matrix, and D is the desired therapeutically active nanoparticles, dispersed in the carrier matrix C, providing the improved nanocomposite microstructure composition of the invention.

In one form of the invention, the improved nanocomposite microstructure composition is formed by the following general reaction:

A + B + E → C + D

where E is one or more facilitating agents, such as a diluent or a surface stabilizer or any other material which may form part of a tablet for oral administration, or other material required for other specific drug delivery, such as the agents and media listed below under the heading *Medicinal and Pharmaceutical Compositions,* or any combination thereof, and the carrier matrix C may thus comprise one or more facilitating agents.

In one form of the invention, co-reactant B is provided in stoichiometric excess of the precursor compound A, and the carrier matrix C comprises unreacted co-reactant B.

In one form of the invention, the carrier matrix C comprises a combination of unreacted co-reactant B and one or more facilitating agents E.

The carrier matrix C may comprise one or more by-products of the reaction between the pre-cursor compound A and the co-reactant B. Thus, the carrier matrix C may comprise one or more of: unreacted co-reactant B, one or more facilitating agents E and one or more by-products of precursor compound A and the co-reactant B.

The carrier matrix C may comprise one or more by-products of the reaction between the pre-cursor compound A and/or the co-reactant B with a facilitating agent E.

Thus, the carrier matrix C may comprise one or more of: unreacted co-reactant B, one or more facilitating agents E, one or more by-products of precursor compound A and the co-reactant B and/or one or more by-products of the reaction between the pre-cursor compound A and/or the co-reactant B with a facilitating agent E.

Preferably, any by-products are non-toxic and/or, if required, readily substantially separable from the improved nanocomposite microstructure composition C+D.

Those knowledgeable in the field will appreciate that there may be many alternate possibilities for the reaction. For instance more than one by-product may be produced.

### 1. Precursor Compounds (A)

Precursor compounds suitable for use in the present invention include any compounds that are capable of reacting with a co-reactant in a solid state mechanochemical reaction to yield a therapeutically active agent capable of existing in nanoparticle form and which participate in a chemical reaction resulting in the production of an active therapeutic agent and reaction by-products that may form part of an inert carrier matrix or a easily substantially removable by-product such as gas, highly volatile liquid, or substance with physical chemical properties which allow for easy separation (e.g. differences in solubility). Note that the by-products may, but do not necessarily have to, contribute an element to the carrier (e.g., CO₂ or H₂O may be by-products which are simply eliminated as gasses). The precursor compound is ordinarily a pharmaceutical compound for which one of skill in the art desires improved properties including smaller particle sizes. The precursor compound is usually a conventional active agent or drug, although the process of the invention may be employed on formulations of agents which already have reduced particle size compared to their conventional parent agent.

Conveniently, the precursor compound of interest is capable of withstanding temperatures that are typical in uncooled mechanochemical processing, which may reach temperatures greater than 80°C. Therefore, compounds with a melting point about 80°C or greater are suitable. For precursor compounds with lower melting points, the reaction vessel may be cooled, thereby allowing compounds with significantly lower melting temperatures to be processed. For instance, a simple water-cooled mill will keep temperatures below 50°C, or chilled water could be used to further lower the milling temperature. Those skilled in the art will understand that a reaction mill could be designed to run at any temperature between say -190 to 500°C. For some reactions it may be advantageous to control the milling temperature to temperatures significantly below the melting points of the precursor compounds and/or co-reactants.

Precursor compounds used in the invention can be, for example, a pharmaceutical agent, including biologics, amino acids, proteins, peptides, nucleotides, nucleic acids, and analogs thereof. The precursor compound can be selected from a variety of known classes of drugs, including, but not limited to: anti-obesity drugs, central nervous system stimulants, carotenoids, corticosteroids, elastase inhibitors, anti-fungals, oncology therapies, anti-emetics, analgesics, cardiovascular agents, anti-inflammatory agents, such as NSAIDs and COX-2 inhibitors, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives (hypnotics and neuroleptics), astringents, alpha-adrenergic receptor blocking agents, beta-adrenoceptor blocking agents, blood products and substitutes, cardiac inotropic agents, contrast media, corticosteroids, cough suppressants (expectorants and mucolytics), diagnostic agents, diagnostic imaging agents, diuretics, dopaminergics (antiparkinsonian agents), haemostatics, immunological agents, lipid regulating agents, muscle relaxants, parasympathomimetics, parathyroid calcitonin and biphosphonates, prostaglandins, radio-pharmaceuticals, sex hormones (including steroids), anti-allergic agents, stimulants and anoretics, sympathomimetics, thyroid agents, vasodilators, and xanthines.

A description of these classes of active agents and a listing of species within each class can be found in Martindale's The Extra Pharmacopoeia, 31st Edition (The Pharmaceutical Press, London, 1996), specifically incorporated by reference. Another source of active agents is the Physicians Desk Reference (60th Ed., pub. 2005), familiar to those of skill in the art. The active agents are commercially available and/or can be prepared by techniques known in the art.

An exhaustive list of drugs for which the methods of the invention are suitable would be burdensomely long for this specification, however, reference to the general pharmacopoeia listed above would allow one of skill in the art to select virtually any drug to which the method of the invention may be applied. Notwithstanding the general applicability of the method of the invention, more specific examples of precursor compounds include, but are not limited to: haloperidol (dopamine antagonist), DL isoproterenol hydrochloride (β-adrenergic agonist), terfenadine (H1-antagonist), propranolol hydrochloride (β-adrenergic antagonist), desipramine hydrochloride (antidepressant), salmeterol (b2-selective adrenergic agonist), sildenafil citrate, tadalafil and vardenafil. Minor analgesics (cyclooxygenase inhibitors), fenamic acids, Piroxicam, Cox-2 inhibitors, and Naproxen, and others, may all benefit from being prepared in a nanoparticle composition. In addition, some active agents may have the benefit of absorption through the skin if presented in a nanoparticle formulation. Such drugs include, but are not limited to, Voltaren (diclofenac), rofecoxib, and Ibuprofen.

Other precursor compounds may be used to prepare nanoparticulate microstructures for treating migraines and other psychotropic disorders, including, for example, 5-hydroxytryptamine receptor antagonists (e.g. ondanstetron, sumatriptan, naratryptan), ergotamine tartrate plus caffeine, or methylsegide.

### 2. Co-reactants (B)

The co-reactants suitable for use in the present invention are chosen primarily for their applicability to, and the nature of, the precursor compound used in the reaction. For example, if the precursor compound is a base, an appropriate co-reactant may be a pharmaceutically acceptable acid. If the precursor compound is an acid, an appropriate co-reactant may be a pharmaceutically acceptable base. If the precursor compound is a salt, an appropriate co-reactant may be an acid or a base. In this respect, the choice of the co-reactant will typically be based on the following considerations.

### (1) Mechanical properties that facilitate the formation of the nano scale microstructures during processing.

A low hardness (typically a Mohs Hardness less than 7) of the solid-state precursor compound is desirable to ensure fracture of the particles during processing, so that nanocomposite microstructures develop during milling.

### (2) Low abrasivity.

Low abrasivity is desirable to minimise contamination of the product powder by the grinding balls (i.e., the milling medium) and mill container. An indirect indication of the abrasivity can be obtained by measuring the level of milling-based contaminants.

### (3) Thermal characteristics.

The precursor compound should be converted to a therapeutically active agent at relatively low temperatures so as to reduce the possibility of thermal degradation. The actual temperature will depend on the properties of the selected precursor compound.

### (4) Agglomeration tendency.

The co-reactant should have a low tendency to agglomerate during milling, particularly in the presence of small amounts of water. It is difficult to quantify the tendency to agglomerate during milling, however, it is possible to obtain a subjective measure by observing the level of "caking" of the co-reactant on the milling media and the mill container as milling progresses.

### (5) No reactivity with nanoparticle compositions.

Preferably, the co-reactant does not react with the nanoparticles (D) in the nanoparticle composition (C+D) during any stage of the process.

### (6) Solubility characteristics.

In circumstances where it is desirable to remove at least a portion of the co-reactant, the co-reactant is preferably highly soluble in biological fluids and / or common solvents such as water or alcohol to facilitate its delivery or selective removal. Preferably still, the co-reactant is soluble in biological fluids and / or common solvents such as water or alcohol to at least 10mg/ml.

In one embodiment, the co-reactant is an inorganic or organic compound selected from the following groups: sodium hydrogen sulfate, sodium hydrogen carbonate, sodium hydroxide, or succinic acid; crystalline organic acids which are pharmaceutically acceptable for salt formation with basic drug entities, for example (but not limited to) fumaric acid, maleic acid, tartaric acid, citric acid); alternatively ammonium salts (or salts of volatile amines) with pharmaceutically acceptable strong inorganic acids for salt formation with basic drugs, for example (but not limited to) ammonium chloride, methylamine hydrochloride, ammonium bromide, when used under conditions under which the ammonia or volatile amine is removed as a gas during the reaction; alternatively where the pharmaceutically acceptable divalent strong inorganic acid (e.g. sulphuric acid) is used as a proton donor during the reaction, the monosodium, potassium, or lithium salt can be used; crystalline hydroxides, hydrogen carbonates, hydrogen carbonates of pharmaceutical acceptable alkali metals, such as but not limited by, sodium, potassium, lithium, calcium, and barium.

In some embodiments of the invention, with particular precursor-co-reactant combinations, the co-reactant is present in an amount such that the resulting carrier matrix accounts for at least 50%, preferably at least 60%, preferably at least 70%, and more preferably at least 80% by volume of the resultant products. This may be desirable to avoid agglomeration. In some embodiments of the invention, with particular precursor-co-reactant combinations, a lower volume of matrix carrier will still act to prevent agglomeration, and in some cases, the matrix carrier can be substantially entirely removed and still permit nanoparticle stability. However, for those embodiments where agglomeration occurs at lower matrix carrier volume fractions, if the stoichiometric reaction described above does not result in a carrier (C) with a volume of at least 80% of the total products, then it may be necessary to introduce an excess of the co-reactant, or one or more facilitating agents, E, at the outset of MCS milling in order to yield the desired nanoparticles.

Co-reactants are selected on the basis of producing a non-toxic carrier and or a desire to be able to substantially remove the carrier to leave substantially pure drug in nanoparticle form. The variations are limited only by one's knowledge of chemistry.

As a matter of illustrating the invention, the following table presents a few examples of preferred precursor compound and co-reactant combinations suitable for use in the method of the invention. The invention is not however limited to these combinations as the disclosure provided herein presents all the information required to select alternate combinations.

**Table 1: Preferred precursor compound / co-reactant combinations**

| **Precursor compound** | **Co-reactant** |
|---|---|
| diclofenac acid | sodium carbonate |
| diclofenac sodium salt | ammonium chloride |
| diclofenac sodium salt | sodium hydrogen sulphate |
| naproxen sodium salt | ammonium chloride |
| naproxen acid | sodium carbonate |
| Olanzapine (free base) | ammonium chloride |
| Ibuprofen sodium salt/free acid | sodium hydrogen sulphate/sodium carbonate |
| Salbutamyl | ammonium chloride |
| naratriptan (base) | ammonium chloride |
| deseril (methyl sergide) sumatriptan | succinic acid |
| viagra (sildenafil free base) | citric acid |

### 3. By-products

When present, depending on the combination of the precursor compound A and the co-reactant B and/or any facilitating agent E, the by-products may include sodium chloride, sodium hydrogencarbonate ammonia, carbon dioxide, water vapor and other gases easily removed. In circumstances where it is desirable to remove at least a portion of the by-product, the by-product is either preferably highly soluble in biological fluids and / or common solvents such as water or alcohol to facilitate its delivery or selective removal or has a sublimation temperature substantially lower than the melting point of the nanoparticles. Preferably still, the by-product is soluble in biological fluids and / or common solvents such as water or alcohol to at least 10mg/ml.

### 4. Facilitating agents (E)

Where it is desirable to prevent agglomeration of the desired therapeutically active nanoparticles (D) of the improved nanocomposite microstructure composition (C+D), facilitating agents such as diluents and surface stabilizers or combinations thereof may be used.

When present, facilitating agents may include surface stabilizers such as CTAB, cetyl pyridinium chloride, gelatin, casein, phosphatides, dextran, glycerol, gum acacia, cholesterol, tragacanth, stearic acid, stearic acid esters and salts, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers, poloxamines, a charged phospholipid, dimyristoyl phophatidyl glycerol, dioctylsulfosuccinate, dialkylesters of sodium sutfosuccinic acid, dioctyl sodium sulfosuccinate, sodium lauryl sulfate, alkyl aryl polyether sulfonates, mixtures of sucrose stearate and sucrose distearate, triblock copolymers of the structure: -(-PEO)-(-PBO-)-(-PEO-)-, p-isononylphenoxypoly-(glycidol), decanoyl-N-methylglucamide; n-decyl β-D-glucopyranoside, n-decyl β-D-maltopyranoside, n-dodecyl β-D-glucopyranoside, n-dodecyl β-D-maltoside, heptanoyl-N-methylglucamide, n-heptyl-β-D-glucopyranoside, n-heptyl β-D-thioglucoside, n-hexyl β-D-glucopyranoside, nonanoyl-N-methylglucamide, n-noyl β-D-glucopyranoside, octanoyl-N-methylglucamide, n-octyl-β-D-glucopyranoside, octyl β-D-thioglucopyranoside, lysozyme, a PEG derivatized phospholipid, PEG derivatized cholesterol, a PEG derivatized cholesterol derivative, PEG derivatized vitamin A, PEG derivatized vitamin E, and random copolymers of vinyl acetate and vinyl pyrrolidone,. and/or mixtures of any of the foregoing. Facilitating agents may also include at least one cationic surface stabilizer selected from the group consisting of a polymer, a biopolymer, a polysaccharide, a cellulosic, an alginate, a nonpolymeric compound, and a phospholipid. Facilitating agents may also include at least one surface stabilizer selected from the group consisting of cationic lipids, benzalkonium chloride, sulfonium compounds, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyltrimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-diclecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10™, tetrabutylammonium bromide, benzyl trimethylammonium bromide, choline esters, benzalkonium chloride, stearalkonium chloride compounds, cetyl pyridinium bromide, cetyl pyridinium chloride, halide salts of quatemized polyoxyethylalkylamines, MIRAPOL™, ALKAQUAT™, alkyl pyridinium salts; amines, amine salts, amine oxides, imide azolinium salts, protonated quaternary acrylamides, methylated quaternary polymers, cationic guar, polymethylmethacrylate trimethylammonium bromide, polyvinylpyrrolidone-2-dimetbylaminoethyl methacrylate dimethyl sulfate, hexadecyltrimethyl ammonium bromide, poly (2-methacryloxyethyltrimethylammonium bromide) (S1001), poly(N-vinylpyrrolidone/2-dimethylaminoethyl methacrylate) di methylsulphate quarternary (S1002), and poly(2-methylacryloxyamidopropyltrimethylammonium chloride) (S1004).

When present, facilitating agents may include, but are not limited to, diluents such as binding agents, filling agents, lubricating agents, sweetners, flavouring agents, preservatives, buffers, wetting agents, disintegrants, effervescent agents, etc.,and/or mixtures of any of the foregoing.

In one form of the invention, a diluent is provided in the form of one or more of the media or agents described below under the heading *"Medicinal and pharmaceutical compositions".*

### 5. Improved nanoparticle compositions (C+D)

The improved nanoparticle compositions directly produced by the method of the present invention comprise nanoparticles of drug (active agent) dispersed in a carrier matrix (after which the carrier matrix may, in some circumstances, be at least partially removed). The nanoparticles of drug have an average particle size measured in diameter as less than 200 nm, preferably less than 100 nm, preferably less than 75 nm, more preferably less than 50 nm, and in some cases less than 30 nm. Preferably, the nanoparticles are distributed in size so that at least 50% of the nanoparticles have a size within the average range, more preferably at least 60%, more preferably at least 70%, and still more preferably at least 75% of the nanoparticles have a size within the average range.

The nanoparticles dispersed within the carrier matrix have advantages over conventional drugs due to their increased surface area and decreased particle size, which may provide increased rate of dissolution, increased absorption, increased bioavailability, and other advantages.

### 6. Distinguishing Precursor compound (A) from Product (D)

Once an improved nanocomposite microstructure composition has been formed in accordance, one may need to distinguish product (D) from the precursor compound (A). Ideally, A and D may be distinguished from each other by changes in a measurable physical property, such as melting point, solubility or size using techniques known to those skilled in the art. Other analytical techniques for distinguishing the precursor compound from product include differential scanning calorimetry (DSC), X-ray diffraction (XRD) or Infrared spectroscopy (IRS).

Particle size of the nanocomposite microstructure composition may be determined by methods known to those skilled in the art. Such techniques include, for example, disk centrifugation, static light scattering, dynamic light scattering or sedimentation field flow fractionation.

### Medicinal and pharmaceutical compositions

The present invention also provides medicinal and pharmaceutical compositions produced using the nanocomposite microstructure composition of the invention. Such compositions may include the composition alone (in the form of C+D, or where some or all of the carrier matrix has been removed), or more preferably the composition may be combined with one or more pharmaceutically acceptable carriers, as well as other agents commonly used in the preparation of pharmaceutically acceptable compositions.

As used herein "pharmaceutically acceptable carrier" includes any and all solvents, dispersion media, coatings, antibacterial and antifungal agents, isotonic and absorption delaying agents, and the like that are physiologically compatible. Preferably, the carrier is suitable for parenteral administration, intravenous, intraperitoneal, intramuscular, sublingual, transdermal or oral administration. Pharmaceutically acceptable carriers include sterile aqueous solutions or dispersions and sterile powders for the extemporaneous preparation of sterile injectable solutions or dispersion. The use of such media and agents for the manufacture of pharmaceutical compositions is well known in the art. Except insofar as any conventional media or agent is incompatible with the nano-particular preparation, use thereof in the manufacture of a pharmaceutical composition according to the invention is contemplated.

Pharmaceutical compositions according to the invention may include one or more of the following examples:
(1) polymeric surface stabilizers which are capable of adhering to the surface of the active agent but do not take part in or undergo any chemical reaction with the active agent itself, such as polymeric surface stabilizers, including, but are not limited to polyethylene glycol (PEG), polyvinylpyrrolidone (PVP), polyvinylalcohol, corspovidone, polyvinylpyrrolidone-polyvinylacytate copolymer, cellulose derivatives, hydroxypropylmethyl cellulose, hydroxypropyl cellulose, carboxymethylethyl cellulose, hydroxypropyllmethyl cellulose phthalate, polyacrylates and polymethacrylates, urea, sugars, polyols, and their polymers, emulsifiers, sugar gum, starch, organic acids and their salts, vinyl pyrrolidone and vinyl acetate; and or
(2) binding agents such as various celluloses and cross-linked polyvinylpyrrolidone, microcrystalline cellulose; and or
(3) filling agents such as lactose monohydrate, lactose anhydrous, and various starches; and or
(4) lubricating agents such as agents that act on the flowability of the powder to be compressed, including colloidal silicon dioxide, talc, stearic acid, magnesium stearate, calcium stearate, silica gel; and or
(5) sweeteners such as any natural or artificial sweetener including sucrose, xylitol, sodium saccharin, cyclamate, aspartame, and accsulfame K; and or
(6) flavouring agents; and or
(7) preservatives such as potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride; and or
(8) buffers; and or
(9) Diluents such as pharmaceutically acceptable inert fillers, such as microcrystalline cellulose, lactose, dibasic calcium phosphate, saccharides, and/or mixtures of any of the foregoing; and or
(10) wetting agents such as com starch, potato starch, maize starch, and modified starches, croscarmellose sodium, crosspovidone, sodium starch glycolate, and mixtures thereof; and or
(11) disintegrants; and or
(12) effervescent agents such as effervescent couples such as an organic acid (eg, citric, tartaric, malic, fumaric, adipic, succinic, and alginic acids and anhydrides and acid salts), or a carbonate (eg sodium carbonate, potassium carbonate, magnesium carbonate, sodium glycine carbonate, L-lysine carbonate, and arginine carbonate) or bicarbonate (eg sodium bicarbonate or potassium bicarbonate); and or
(13) other pharmaceutically acceptable excipients.

Pharmaceutical compositions suitable for use in animals and in particular in man typically must be sterile and stable under the conditions of manufacture and storage. The pharmaceutical composition comprising nanoparticles can be formulated as a solution, microemulsion, liposome, or other ordered structure suitable to high drug concentration. Actual dosage levels of the therapeutically active agent in the pharmaceutical composition of the invention may be varied in accordance with the nature of the therapeutically active agent, as well as the potential increased efficacy due to the advantages of providing and administering the active agent in nanoparticle form (e.g., increased solubility, more rapid dissolution, increased surface area of the drug in nanoparticle form, etc.). Thus as used herein "therapeutically effective amount" will refer to an amount of nanocomposite microstructure composition required to effect a therapeutic response in an animal. Amounts effective for such a use will depend on: the desired therapeutic effect; the route of administration; the potency of the therapeutically active agent; the desired duration of treatment; the stage and severity of the disease being treated; the weight and general state of health of the patient; and the judgment of the prescribing physician.

### Mode of administration of pharmaceutical compositions comprising nanoparticle formulations

Pharmaceutical compositions of the invention can be administered to humans and animals in any pharmaceutically acceptable manner, such as orally, rectally, pulmonary, intravaginally, locally (powders, ointments or drops), transdermal, or as a buccal or nasal spray.

Solid dosage forms for oral administration include capsules, tablets, pills, powders, pellets, and granules. For capsules, tablets, and pills, the dosage forms may also comprise buffering agents. Further, incorporating any of the normally employed excipients, such as those previously listed, and generally 10-95% of the nanoparticulate preparation, and more preferably at a concentration of 25%-75% will form a pharmaceuticaffy acceptable non-toxic oral composition.

Pharmaceutical compositions of the invention may be parenterally administered as a solution of the nanocomposite microstructure composition suspended in an acceptable carrier, preferably an aqueous carrier. A variety of aqueous carriers may be used, e.g., water, buffered water, 0.4% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration.

For aerosol administration, pharmaceutical compositions of the invention are preferably supplied along with a surface stabilizer and propellant. The surface stabilizer must, of course, be non-toxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surface stabilizer may constitute 0.1 %-20% by weight of the composition, preferably 0.25-5%. The balance of the composition is ordinarily propellant. A carrier can also be included, as desired, as with, e.g., lecithin for intranasal delivery.

Pharmaceutical compositions of the invention may also be administered via liposomes, which serve to target the active agent to a particular tissue, such as lymphoid tissue, or targeted selectively to cells. Liposomes include emulsions, foams, micelles, insoluble monolayers, liquid crystals, phospholipid dispersions, lamellar layers and the like. In these preparations the nanocomposite microstructure composition is incorporated as part of a liposome, alone or in conjunction with a molecule that binds to or with other therapeutic or immunogenic compositions.

### Therapeutic uses

The present invention is further directed to methods of treatment of an animal comprising administering to said animal a therapeutically effective amount of a composition comprising a nanocomposite microstructure composition produced according to a method of the invention.

Some key therapeutic uses of the improved nanocomposite microstructure compositions of the invention include pain relief, anti-inflammatory, migraine, asthma, and other disorders that require the active agent to be administered with a high bioavailability.

One of the main areas when rapid bioavailability of an active agent is required is in the relief of pain. The minor analgesics, such as cyclooxgenase inhibitors (aspirin related drugs) may be prepared as nanocomposite microstructure compositions according to the present invention.

Improved nanocomposite microstructure compositions of the invention may also be used for treatment of eye disorders. That is, the composition may be formulated for administration on the eye as an aqueous suspension in physiological saline, or a gel. In addition, the composition may be prepared in a powder form for administration via the nose for rapid central nervous system penetration.

Treatment of cardiovascular disease may also benefit from active agents prepared as nanocomposite microstructure compositions according to the invention. For example, active agents used for treatment of angina pectoris. In particular molsidomine may benefit from better bioavailability.

Other therapeutic uses of the nanocomposite microstructure compositions of the present invention include treatment of hair loss, sexual dysfunction, or dermal treatment of psoriasis.

### Purified nano-particulate therapeutically active agent

Another embodiment of the invention relates to a method for preparing a purified nanoparticulate therapeutically active agent comprising the steps of:
(i) contacting a precursor compound with a co-reactant under mechanochemical synthesis conditions to generate a solid-state chemical reaction between the precursor compound and the co-reactant to produce therapeutically active nanoparticles dispersed in a carrier matrix; and
(ii) removing at least a portion of the carrier matrix to release the therapeutically active nanoparticles.

The step of removing at least a portion of the carrier matrix to release the therapeutically active nanoparticles may comprise the step of dissolving or subliming the carrier matrix to release the therapeutically active nanoparticles.

The step of dissolving the carrier matrix may involve subjecting the product of step (i) to a suitable solvent that selectively removes the carrier, while not reacting with the therapeutically active nanoparticles. In a highly preferred embodiment, the carrier matrix is removed by washing with a solvent, followed by rinsing with water. Appropriate solvents may be acid, alkaline or neutral aqueous solutions, or any suitable organic solvent. This may be any solvent in which the drug is insoluble but the matrix is soluble or alternatively in which the drug and matrix can be separated by differential centrifugation. For example, where the carrier matrix is formed from sodium chloride a suitable solvent would be any aqueous medium like water. Removal of the carrier matrix will leave substantially purified therapeutically active nanoparticles. Alternatively, it may be possible to sublimate the carrier from the nanoparticles.

In some cases (see, e.g, Example 2 below), the nanoparticle composition resulting from removal of the matrix carrier may require stabilization with a surface stabilizer. Example surface stabilizers include those listed above; in some cases the preferred stabilizer is CTAB,. Those of skill in the art will appreciate that a wide variety of other surface stabilizers are suitable for such stabilization.

In some cases, the method for removal of the matrix carrier and surface stabilization may cause a chemical reversion of the nanoparticle composition. For example, in Example D below, the nanoparticle composition diclofenac sodium salt stabilized in sodium carbonate carrier matrix, reverts to a nanoparticle composition of diclofenac acid. The nanoparticles remain, thus this reversion will be useful when the desired nanoparticle formulation is one comprising nanoparticles of the conventional precursor starting material.

Should additional purification be required, then conventional purification techniques may be employed to separate therapeutically active nanoparticles from any contaminants. The appropriate technique will depend on the nature of the purification required. Those skilled in the art are familiar with such techniques and would readily appreciate adaptation of such techniques to the nanoparticle formulations of the invention.

The invention also includes the product of the aforementioned method and its use in the preparation of medicaments and pharmaceutical compositions suitable for treating an animal. The invention thus includes methods for preparing medicaments and pharmaceutically acceptable compositions comprising the purified nanoparticulate therapeutically active agent.

The present invention will now be described with reference to the following nonlimiting Examples. The description of the Examples is in no way limiting on the preceding paragraphs of this specification, but is provided for exemplification of the methods and compositions of the invention.

### Examples

### A: Mechanochemical processing of diclofenac sodium with an excess of sodium hydrogen sulfate

Mechanochemical processing of diclofenac sodium with an excess of sodium hydrogen sulfate has been used to manufacture ultra-fine (less than 1000nm) particles of diclofenac acid. In this particular example, the sodium hydrogen sulfate reagent acts as both a chemical reactant and diluent.

A reactant mixture consisting of 1.00g of diclofenac sodium and 9.00g of sodium sulfate was milled for 6 hours using a Spex 8000 mixer/mill with twenty 9.5 mm stainless steel balls as the grinding media. This resulted in the formation of a nano-crystalline powder consisting of ultra-fine diclofenac particles, sodium sulfate, and sodium hydrogen sulfate.

Ultra-fine particles of diclofenac acid were recovered by removing the inorganic salts through washing with dilute hydrochloric acid followed by rinsing with deionised water. The washed powder was subsequently dried at 50oC for several hours in air.

Five point BET analysis of the final diclofenac acid powder gave a specific surface area of 8.9 m2/g, corresponding to an average particle size of around 450nm.

### B: Mechanochemical processing of diclofenac sodium with sodium hydrogen sulfate in the presence of sodium sulfate

Mechanochemical processing of diclofenac sodium with sodium hydrogen sulfate in the presence of sodium sulfate has been used to manufacture ultra-fine particles of diclofenac acid. In this example, the sodium sulfate reagent acts solely as an inert diluent.

A reactant mixture consisting of 1.07g of diclofenac sodium, 0.44g of sodium hydrogen sulfate and 8.49g of sodium sulfate was milled for 6 hours using a Spex 8000 mixer/mill with twenty 9.5 mm stainless steel balls as the grinding media. This resulted in the formation of a nano-crystalline powder consisting of ultra-fine diclofenac acid particles and sodium sulfate.

The ultra-fine particles of diclofenac acid were recovered by removing the sodium sulfate through washing with dilute hydrochloric acid followed by rinsing with deionised water. The washed powder was subsequently dried at 50°C for several hours in air.

Five point BET analysis of the final diclofenac acid powder gave a specific surface area of 5.8 m²/g corresponding to an average particle size of about 640nm.

### C. Mechanochemical processing of diclofenac acid plus excess carbonate

Mechanochemical processing of diclofenac acid with an excess of carbonate was used to manufacture ultra-fine particles of diclofenac sodium dispersed within a matrix of sodium carbonate.

A reactant mixture consisting of 1.50g of diclofenac acid and 8.50g of sodium carbonate was milled for 6 hours using a Spex 8000 mixer/mill with twenty 9.5 mm stainless steel balls as the grinding media. This resulted in the formation of a nano-crystalline powder consisting of ultra-fine diclofenac sodium particles dispersed within a matrix of sodium carbonate.

### Results

Differential scanning calorimetry (DSC) and X-Ray diffraction (XRD) were used to analyse reaction products. Using DSC, sodium salt and acid forms of diclofenac exhibit different behaviours during heating. The sodium salt shows no change until undergoing a decomposition reaction at around 275°C. In contrast, the acid form shows two thermal events beginning around 140°C and a continuous mass loss due to sublimation. XRD patterns for acid and salt forms are well documented in the litereature.

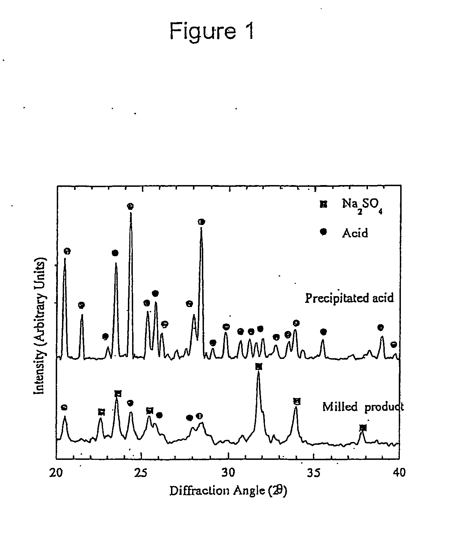

Figure 1 shows XRD traces of (a) precipitated diclofenac and (b) a stoichiometric mixture of diclofenac sodium and sodium hydrogen sulfate that had been milled for 6 hours. The pattern of the milled powder contains diffraction peaks corresponding to the expected reaction products of sodium sulfate and diclofenac acid. This confirms that the reaction has progressed, with the shorter and broader peaks in the milled reaction indicating a combination of reduced particle size and induction of amorphisation.

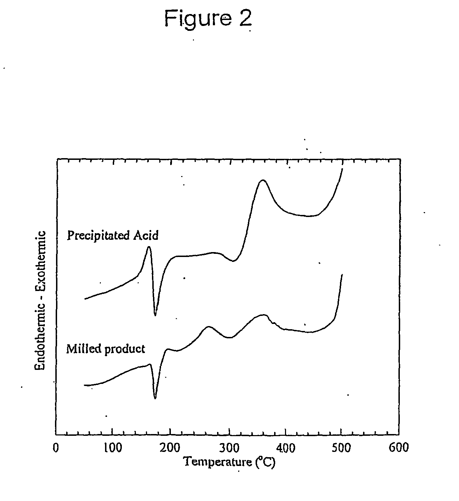

Figure 2 shows TG-DSC traces of precipitated diclofenac acid and milled diclofenac sodium (2.90g) plus sodium hydrogen sulfate (1.18g). As shown, the thermal behaviour of these two samples was different, however the presence of the typical thermal decomposition profile of diclofenac acid in the milled reaction can be clearly seen confirming the progression of the reaction. These differences could be attributed to presence of sodium sulfate, which may delay the decomposition of the diclofenac acid by acting as a diffusion barrier.

### Example D: Nanoparticle composition of diclofenac sodium salt dispersed in Na₂CO₃

A nanoparticle composition of diclofenac sodium salt dispersed in a carrier matrix comprising Na₂CO₃ was prepared as follows:
The precursor compound, 0.441 g of conventional diclofenac acid powder, 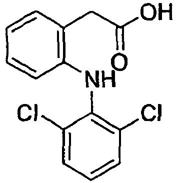 was placed in a milling apparatus (a 70 cm³ stainless steel ball mill container) with an excess (4.3 g) of the co-reactant, Na₂CO₃, thereby providing the mixture of reactants at 9 and 91 weight % respectively, corresponding to 15 and 85 volume %, with a total volume of 2 cm³. Milling media comprising 10g of 10mm chrome steel balls (10 pieces) were employed in the container. The composition resulting after MCS comprised nanoparticles of diclofenac sodium salt (10.0 wt.%, 16 vol.%), 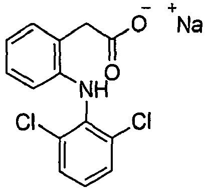 dispersed in a carrier matrix of Na₂CO₃ (87.4% wt.%, 81.1 vol.%) and NaHCO₃ (2.6 wt.%, 2.9 vol.%).

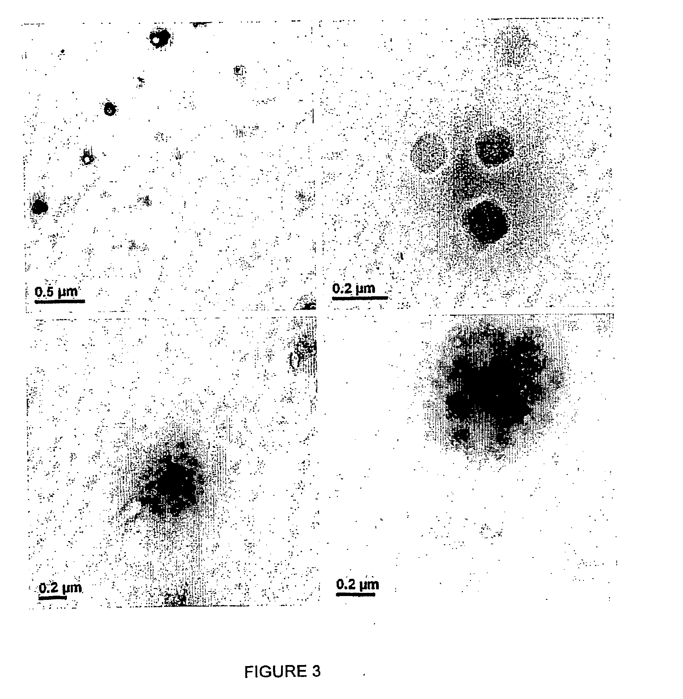

Transmission electron microscopy (TEM) of the resulting nanoparticle composition (dispersed in hexane for convenience), showed nanoparticles less than 200nm (on the order of 30-50 nm), as shown in Fig. 3.

In order to examine the effect of milling time, IR analysis was carried out on the initial reactants (i.e., MCS for 0 minutes), as well as 5, 15, and 90 minutes. While the 90 minute run yielded the highest degree of reaction to produce the salt form of diclofenac, more than about 90% of the nanoparticle diclofenac salt was formed within 15 minutes.

In two separate MCS runs, milling media comprised steel balls of 10 and 2 mm, respectively. IR-spectra analysis revealed that the 10mm steel balls performed better than the 2 mm steel balls with this combination of reactants, as the 2 mm balls yielded an undesirable degree of caking. This IR analysis was sufficiently sensitive to show exceedingly small amounts of diclofenac acid. An experiment was performed with mixtures of diclofenac acid and salt in the carrier matrix keeping the total diclofenac percentage in matrix to 15% wt.%. IR analysis on varying amounts of the acid and salt in the mixture revealed the presence of the acid in amounts as little as 1.5 wt.%.

DSC analysis of the nanoparticle composition's melting point confirmed that the resulting composition was diclofenac sodium salt, with a melting point in the range of 275-285 °C. This was true even in the 5 minute MCS run.

In order to examine the effect of removing the matrix from the nanoparticle composition, and whether the nanoparticle composition would remain as diclofenac sodium salt or revert to diclofenac acid, the following washing steps were undertaken. 5 mg of sample after MCS was mixed with 1 ml of 0.5 M HCl and 1 mM CTAB (Cetyl Trimethyl Ammonium Bromide) in a centrifugation tube (heeding caution for the development of CO₂, working with safety glasses when opening the lid of the tube). The sample was vortexed for 2 minutes, followed by ultrasound treatment for 5 seconds, followed by 1 minute additional vortexing. The sample was then subjected to 4 repeats of: centrifugation (3,000 g, 3 minutes), removal of supernatant, addition of 0.01 M HCl and 1 mM CTAB, and redispersion by vortex and ultrasound sonication (30 seconds each).

The resulting nanoparticle composition, stabilized with the surface stabilizer CTAB, was found to have reverted to the diclofenac acid form of the drug, with nanoparticles on the order of 100-200 nm. XRD analysis, melting point analysis, and IR-spectra analysis all independently confirmed that the nanoparticle composition comprised diclofenac acid, rather than the sodium salt thereof.

### Example E: Nanoparticle composition of diclofenac acid dispersed in NH₄Cl

A nanoparticle composition of diclofenac acid dispersed in a carrier matrix comprising NH₄Cl was prepared as follows:
The precursor compound, 0.43 g of conventional diclofenac sodium salt powder, 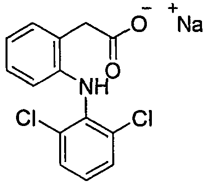
was placed in a milling apparatus (a 70 cm³ stainless steel ball mill container) with an excess (2.6 g) of the co-reactant, NH₄Cl, thereby providing the mixture of reactants at 13.7 and 86.3 weight % respectively, corresponding to 15 and 85 volume %, with a total volume of 2 cm³. Milling media comprising 10g of 10mm steel balls (10 pieces) were employed in the container. Some cooling was achieved by allowing compressed air(100 kcpa) to flow over the milling container . The composition resulting after MCS, after only 15 minutes comprised nanoparticles of diclofenac acid (12.9 wt.%, 13.4 vol.%), 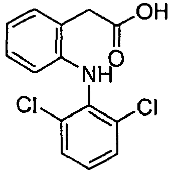 dispersed in a carrier matrix comprising NH₄Cl (84.7% wt.%, 84.8 vol.%) and NaCl (2.5 wt.%, 1.8 vol.%), with a byproduct of ammonia gas.

DSC analysis of the nanoparticle composition's melting point confirmed that the resulting composition was diclofenac acid, with a melting point in the range of 156-165 °C, with no trace of diclofenac sodium salt (melting point of 275-285 °C).

In order to examine the effect of removing the matrix from the nanoparticle composition, and whether the nanoparticle composition would remain as diclofenac acid, the nanoparticle composition was washed as follows to remove the matrix carrier. 5 mg of sample after MCS was mixed with 1 ml of 0.01 M HCl and 1 mM CTAB (Cetyl Trimethyl Ammonium Bromide) in a centrifugation tube (heeding caution for the development of CO2, working with safety glasses when opening the lid of the tube). The sample was vortexed for 2 minutes, followed by ultrasound treatment for 5 seconds, followed by 1 minute additional vortexing. The sample was then subjected to 4 repeats of: centrifugation (3,000 g, 3 minutes), removal of supernatant, addition of 0.01 M HCl and 1 mM CTAB, and redispersion by vortex and ultrasound sonication (30 seconds each).

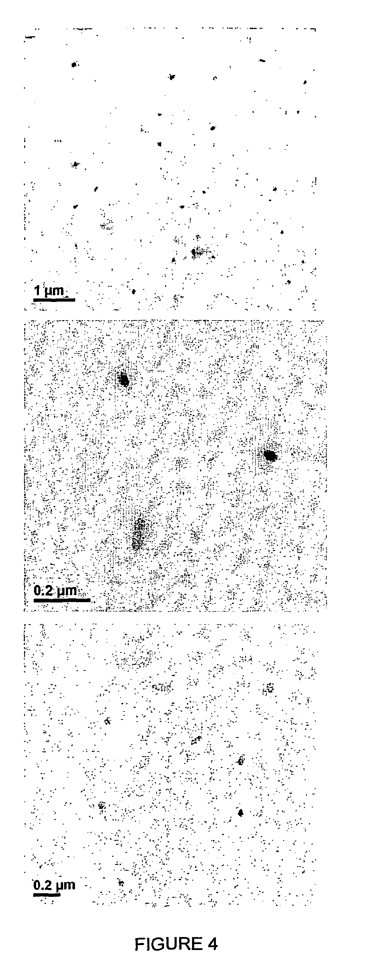

The resulting nanoparticle composition, stabilized with the surface stabilizer CTAB, was found to remain comprising the diclofenac acid form of the drug (by XRD, FTIR and DSC), with nanoparticles less than 200nm and the majority on the order of 30-50 nm. TEM of the washed nanoparticle composition, stabilized with the surface stabilizer CTAB, also showed both spherical and nonspherical nanoparticles, the nonspherical particles appearing to be rod-shaped and have a minor-axis dimension of about 30 nm and a major-axis dimension of about 150 nm. See Fig. 4. It is possible that the appearance of these nonspherical particles is a result of linear agglomeration of individual nanoparticles.

### Example F: Nanoparticle composition of naproxen sodium salt dispersed in Na₂CO₃

A nanoparticle composition of naproxen sodium salt dispersed in a carrier matrix comprising Na₂CO₃ was prepared as follows:

The precursor compound, 0.6014 g of conventional naproxen acid powder, 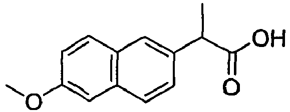 was placed in a milling apparatus (a 70 cm³ stainless steel ball mill container) with an excess (3.401 g) of the co-reactant, Na₂CO₃, thereby providing the mixture of reactants at 15 and 85 weight % respectively, corresponding to 27.4 and 72.6 volume %, with a total volume of 1.85 cm³. Milling media comprising 10g of 10mm steel balls (10 pieces) were employed in the container. Cooling was achieved with compressed air flow (100 kcpa). The composition resulting after MCS comprised nanoparticles of naproxen sodium salt (16.5 wt.%, 26.3 vol.%), 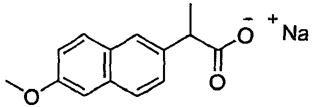 dispersed in a carrier matrix of Na₂CO₃ (68.2% wt.%, 81.1 vol.%) and NaHCO₃ (5.0 wt.%, 5.5 vol.%)

Scanning electron microscopy (SEM) of the resulting nanoparticle composition showed nanocrystalline structures and nanoparticles on the order of 100 nm.

Milling times assayed included 60, 150, and 240 minutes. In two separate MCS runs, milling media comprised steel balls of 10 and 2 mm, respectively. IR-spectra analysis of this nanoparticle composition revealed that the 2mm steel balls performed better than the 10 mm steel balls.

Analysis of the melting point of the nanoparticle composition produced with 2 mm steel balls with a 60 minute MCS time period confirmed that the resulting composition was naproxen sodium salt, with a melting point in the range of 250-260 °C, as opposed to that of naproxen acid, in the range of 150-160 °C. Two other MCS time periods were assayed, 150 and 240 minutes, both of which showed some degradation of the drug. The nanoparticle composition produced with 10mm balls did not reveal the melting point of either naproxen acid or salt, implying that the drug had been degraded even at the shorter time point of 60 minutes.

### Example G: Nanoparticle composition of naproxen acid dispersed in NH₄Cl

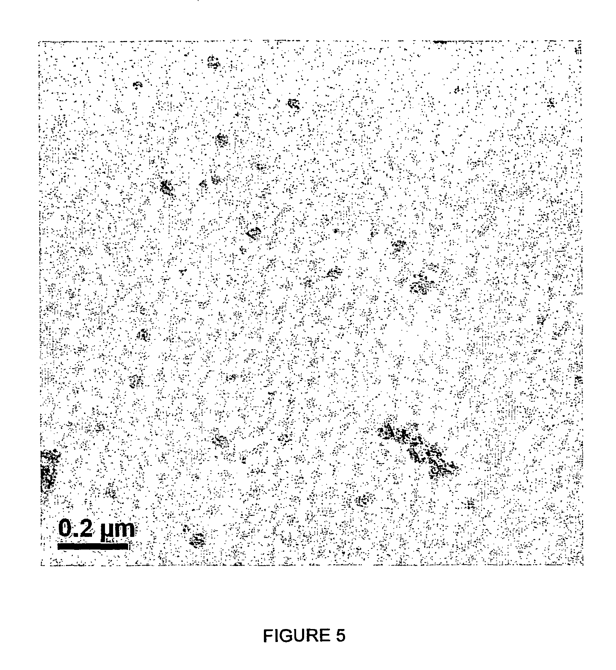

A nanoparticle composition of naproxen acid dispersed in a carrier matrix comprising NH₄Cl was prepared as follows:
The precursor compound, 0.4158 g of conventional naproxen sodium salt powder, 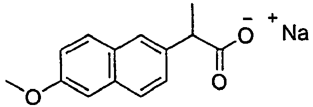 was placed in a milling apparatus (a 70 cm³ stainless steel ball mill container) with an excess (2.5959 g) of the co-reactant, NH₄Cl, thereby providing the mixture of reactants at 13.8 and 86.2 weight % respectively, corresponding to 15 and 85 volume %, with a total volume of 2 cm³. Milling media comprising 10g of 10mm steel balls (10 pieces) were employed in the container. Cooling was achieved with compressed air flow (100 kcpa). The composition resulting after MCS comprised nanoparticles of naproxen acid (12.7 wt.%, 16 vol.%),
dispersed in a carrier matrix of NH₄Cl (84% wt.%, 82 vol.%) and NaCl (3.3 wt.%, 2.0 vol.%) with a byproduct of ammonia gas. 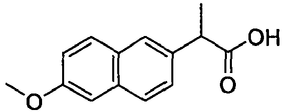 Transmission electron microscopy (TEM) of the resulting nanoparticle composition (dispersed in hexane for convenience), showed nanoparticles on the order of 30-50 nm, as shown in Fig. 5.

In order to examine the effect of milling time, DCS and IR analysis were carried out on the nanoparticle formulations resulting from MCS times of 60, 150, and 240 minutes. Additionally, DSC melting point analysis was performed on the products of MCS times of 5, 10, 15, 30, 60, and 90 minutes.

DSC analysis of the nanoparticle compositions' melting points confirmed that the resulting compositions comprised naproxen acid to a varying degree, the longer MCS times showing degradation, while times of 90 minutes and under showing melting points in the range of 150-160 °C. However, times longer than 30 minutes also showed signs of undesirable impurities (e.g., darkening of powder, melting point reduction).

FTIR analysis confirmed that naproxen acid was present in the nanoparticle compositions produced with MCS times of 60, 150, and 240 minutes (the only times tested in this assay), but the proximity of the fingerprint windows for naproxen acid and the matrix carrier rendered a quantitative analysis of the relative presence of the acid and salt forms impossible.

In order to examine the effect of removing the matrix from the nanoparticle composition, and whether the nanoparticle composition would remain as naproxen acid, the nanoparticle composition was washed as follows to remove the matrix carrier. 5 mg of sample after MCS was mixed with 1 ml of 0.01 M HCl and 1 mM CTAB (Cetyl Trimethyl Ammonium Bromide) in a centrifugation tube (heeding caution for the development of CO2, working with safety glasses when opening the lid of the tube). The sample was vortexed for 2 minutes, followed by ultrasound treatment for 5 seconds, followed by 1 minute additional vortexing. The sample was then subjected to 2 repeats of: centrifugation (3,000 g, 3 minutes), removal of supernatant, addition of 0.1 M HCl and 1 mM CTAB, and redispersion by vortex and ultrasound sonication (30 seconds each).

The resulting nanoparticle composition, stabilized with the surface stabilizer CTAB, was found to remain comprising the naproxen acid form of the drug by melting point analysis, with nanoparticles on the order of 50 nm. TEM of the washed nanoparticle composition, stabilized with the surface stabilizer CTAB, also showed both spherical and nonspherical nanoparticles, the nonspherical particles appearing to have a minor-axis dimension of about 50 nm and a major-axis dimension of about 150 nm. See Fig. 4. It is possible that the appearance of these nonspherical particles is a result of linear agglomeration of individual nanoparticles. XRD of the post-washed material revealed a single phase crystalline product of naproxen acid

Removal of the matrix carrier was also performed on a 60 mg sample of the nanoparticle composition via sublimation in a sublimation apparatus, comprising a vacuum at 0.05-0.01 mm Hg at about 135-140 °C. While the sublimation was successful, the fact that the sample was electrostatically charged rendered removal and subsequent analysis difficult.

### Example H: Nanoparticle composition of olanzapine HCl dispersed in NH₄Cl

A nanoparticle composition of olanzapine HCl dispersed in a carrier matrix comprising NH₄Cl was prepared as follows:
The precursor compound, 0.386g of conventional olanzapine powder (free base), was placed in a milling apparatus (a 70 cm³ hardened steel ball mill container) with an excess 2.596g of the co-reactant, NH₄Cl, thereby providing the mixture of reactants at 13and 87weight % respectively, corresponding to 15 and 85 volume %, with a total volume of 2 cm³. Milling media comprising 10g of 10mm steel balls (10 pieces) were employed in the container. Cooling was achieved with compressed air flow (100 kcpa). MCS times assayed included 0, 5, 15, 30, and 60 minutes. The composition resulting after MCS comprised nanoparticles of olanzapine HCl (14.3 wt.%, 17 vol.%), dispersed in a carrier matrix of NH₄Cl (85 wt.%, 83 vol.%), with a byproduct of ammonia gas.

DSC analysis of the melting point of the nanoparticle compositions produced by MCS confirmed that the resulting composition comprised olanzapine HCl to a varying degree, even as early as 5 minutes. By 15 minutes and longer, a characteristic spike at about 210-220 °C appeared, indicating the presence of olanzapine HCl.

### Example I: Nanoparticle composition of sildenafil citrate dispersed in citric acid matrix

A nanoparticle composition of sildenafil dispersed in a carrier matrix comprising citric acid was prepared as follows. Initially, 2 g of conventional sildenafil citrate was extracted with CH₂Cl₂ (DCM) in the presence of sodium bicarbonate to yield 1.35 g of sildenafil free base and sodium citrate. This sildenafil free base was then used as the precursor compound for the MCS reaction of the invention as follows.

A mixture of the sildenafil free base, 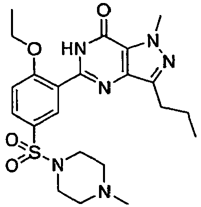 and the co-reactant, citric acid, corresponding to 15 and 85 volume %, with a total volume of 2 cm³, was placed in a milling apparatus (a 70 cm³ stainless steel ball mill container). Milling media comprising 10g of 10mm steel balls (10 pieces) were employed in the container. The composition resulting after MCS may comprise nanoparticles of sildenafil citrate, dispersed in a carrier matrix of citric acid. Milling times included 0, 5, 15, 30, 60, and 90 minutes.

DSC analysis of the nanoparticle compositions' melting points proved difficult as the melting point of decomposing citric acid obscures the expected melting point spike.

It will be apparent to persons skilled in the materials and pharmaceutical arts that numerous enhancements and modifications can be made to the above described processes without departing from the basic inventive concepts. For example, in some applications the precursor pharmaceutically active agent compound may be pretreated and supplied to the process in the pretreated form. All such modifications and enhancements are considered to be within the scope of the present invention, the nature of which is to be determined from the foregoing description and the appended claims. Furthermore, the preceding Examples are provided for illustrative purposes only, and are not intended to limit the scope of the processes or compositions of the invention.

## Claims

1. A method of producing a nanoparticle composition comprising nanoparticles of a therapeutically effective agent, comprising the step of:
mechanochemical synthesis of a mixture of a precursor compound and a co-reactant using milling media in a milling apparatus, for a time period sufficient to produce the nanoparticle composition comprising nanoparticles of the therapeutically effective agent dispersed within a solid carrier matrix, wherein the nanoparticles of the therapeutically effective agent are produced by a solid-state chemical reaction between the precursor compound and the co-reactant.

2. The method of claim 1, wherein the nanoparticles have an average size less than 200 nm, preferably less than 100 nm, more preferably less than 75 nm, more preferably less than 50 nm, more preferably less than 40 nm.

3. The method of any preceding claim, wherein the time period is between 5 minutes and 2 hours, more preferably the time period is between 5 minutes and 1 hour, more preferably the time period is between 5 minutes and 45 minutes, more preferably the time period is between 5 minutes and 30 minutes, more preferably, the time period is between 10 minutes and 20 minutes.

4. The method of any of claims 1-3, wherein the milling media comprises steel balls.

5. The method of claim 4, wherein the steel balls have a diameter of between 1 and 20 mm, more preferably the steel balls have a diameter between 2 and 15 mm, more preferably the steel balls have a diameter of between 3 and 10 mm.

6. The method of any preceding claim wherein the precursor compound is selected from the group consisting of biologics, amino acids, proteins, peptides, nucleotides, nucleic acids, and analogs thereof.

7. The method of any preceding claim, wherein the precursor compound is selected from the group consisting of anti-obesity drugs, central nervous system stimulants, carotenoids, corticosteroids, elastase inhibitors, anti-fungals, oncology therapies, anti-emetics, analgesics, cardiovascular agents, anti-inflammatory agents, such as NSAIDs and COX-2 inhibitors, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives (hypnotics and neuroleptics), astringents, alpha-adrenergic receptor blocking agents, beta-adrenoceptor blocking agents, blood products and substitutes, cardiac inotropic agents, contrast media, corticosteroids, cough suppressants (expectorants and mucolytics), diagnostic agents, diagnostic imaging agents, diuretics, dopaminergics (antiparkinsonian agents), haemostatics, immunological agents, lipid regulating agents, muscle relaxants, parasympathomimetics, parathyroid calcitonin and biphosphonates, prostaglandins, radiopharmaceuticals, sex hormones (including steroids), anti-allergic agents, stimulants and anoretics, sympathomimetics, thyroid agents, vasodilators, and xanthines..

8. The method of claim 7, wherein the precursor compound is selected from the group consisting of haloperidol, DL isoproterenol hydrochloride, terfenadine, propranolol hydrochloride, desipramine hydrochloride, salmeterol, sildenafil citrate, tadalafil, vardenafil, fenamic acids, Piroxicam, Naproxen, Voltaren (diclofenac), rofecoxib, ibuprofren ondanstetron, sumatriptan, naratryptan, ergotamine tartrate plus caffeine, methylsegide, olanzapine.

9. The method of any preceding claim, further comprising the step of removing at least a portion of the solid carrier matrix, wherein the nanoparticles have an average particle size of less than 200 nm.

10. The method of claim 9, wherein at least 25% of the solid carrier matrix is removed, more preferably at least 50% of the solid carrier matrix is removed, more preferably at least 75% of the solid carrier matrix is removed, more preferably wherein substantially all of the solid carrier matrix is removed.

11. A nanoparticle composition comprising nanoparticles of diclofenac acid dispersed in a solid carrier matrix, wherein the nanoparticles have an average size less than 200 nm.

12. The nanoparticle composition of claim 11, wherein the average size is less than 100 nm, preferably the average size is less than 75 nm, more preferably the average size is less than 50 nm, more preferably the average size is less than 30 nm.

13. The nanoparticle composition of claim 11, wherein the solid carrier matrix comprises at least one member selected from the group consisting of Na₂CO₃, NaHCO₃, NH₄Cl, and NaCl.

14. The nanoparticle composition of claim 13, wherein the solid carrier matrix comprises either Na₂CO₃ alone or in combination with NaHCO₃, and the nanoparticles of diclofenac are in the form of diclofenac sodium salt.

15. The nanoparticle composition of claim 13, wherein the solid carrier matrix comprises either NH₄Cl alone or in combination with NaCl, and the nanoparticles of diclofenac are in the form of a diclofenac acid.

16. A nanoparticle composition comprising nanoparticles of naproxen acid dispersed in a solid carrier matrix, wherein the solid carrier matrix comprises either NH₄Cl alone or in combination with NaCl, and the nanoparticles have an average size less than 200 nm.

17. A nanoparticle composition comprising nanoparticles of olanzapine dispersed in a solid carrier matrix, wherein the nanoparticles have an average size less than 200 nm.

18. The nanoparticle composition of claim 17, wherein the average size is less than 100 nm, preferably wherein the average size is less than 75 nm, more preferably wherein the average size is less than 50 nm, more preferably wherein the average size is less than 30 nm.

19. The nanoparticle composition of claim 17, wherein the solid carrier matrix comprises NH₄Cl.

20. The nanoparticle composition of claim 17, wherein the nanoparticles of olanzapine are in the form of olanzapine HCl.

21. A nanoparticle composition comprising nanoparticles of sildenafil dispersed in a solid carrier matrix, wherein the nanoparticles have an average size less than 200 nm.

22. The nanoparticle composition of claim 21, wherein the average size is less than 100 nm, preferably wherein the average size is less than 75 nm, more preferably wherein the average size is less than 50 nm, more preferably wherein the average size is less than 30 nm.

23. The nanoparticle composition of claim 21, wherein the carrier matrix comprises citric acid.

24. The nanoparticle composition of claim 21, wherein the nanoparticles of sildenafil are in the form of sildenafil base.

## Patentansprüche

1. Verfahren zum Herstellen einer Nanopartikelzusammensetzung, die Nanopartikel eines therapeutischen Wirkstoffs umfasst, umfassend die Schritte:
mechanochemische Synthese eines Gemischs einer Vorläuferverbindung und eines Koreaktionsmittels unter Verwendung eines Mahlmediums in einer Mahlvorrichtung über einen Zeitraum, der ausreicht, um die Nanopartikelzusammensetzung, die Nanopartikel des therapeutischen Wirkstoffs in einer festen Trägermatrix dispergiert umfasst, herzustellen, wobei die Nanopartikel des therapeutischen Wirkstoffs durch eine chemische Festkörperreaktion zwischen der Vorläuferverbindung und dem Koreaktionsmittel hergestellt werden.

2. Verfahren gemäß Anspruch 1, wobei die Nanopartikel eine mittlere Größe von weniger als 200 nm aufweisen, vorzugsweise weniger als 100 nm, bevorzugter weniger als 75 nm, bevorzugter weniger als 50 nm, bevorzugter weniger als 40 nm.

3. Verfahren gemäß einem der vorstehenden Ansprüche, wobei der Zeitraum zwischen 5 Minuten und 2 Stunden beträgt, der Zeitraum bevorzugter zwischen 5 Minuten und 1 Stunde beträgt, der Zeitraum bevorzugter zwischen 5 Minuten und 45 Minuten beträgt, der Zeitraum bevorzugter zwischen 5 Minuten und 30 Minuten beträgt, der Zeitraum bevorzugter zwischen 10 Minuten und 20 Minuten beträgt.

4. Verfahren gemäß einem der Ansprüche 1-3, wobei das Mahlmedium Stahlkugeln umfasst.

5. Verfahren gemäß Anspruch 4, wobei die Stahlkugeln einen Durchmesser zwischen 1 und 20 mm aufweisen, die Stahlkugeln bevorzugter einen Durchmesser zwischen 2 und 15 mm aufweisen, die Stahlkugeln bevorzugter einen Durchmesser zwischen 3 und 10 mm aufweisen.

6. Verfahren gemäß einem der vorstehenden Ansprüche, wobei die Vorläuferverbindung ausgewählt ist aus der Gruppe bestehend aus biologischen Stoffen, Aminosäuren, Proteinen, Peptiden, Nukleotiden, Nukleinsäuren und Analoga davon.

7. Verfahren gemäß einem der vorstehenden Ansprüche, wobei die Vorläuferverbindung ausgewählt ist aus der Gruppe bestehend aus Antiadipositas-Arzneimitteln, Stimulationsmitteln für das zentrale Nervensystem, Carotenoiden, Corticosteroiden, Elastasehemmern, antifungiellen Mitteln, Onkologietherapien, Antiemetika, Analgetika, Herz-Kreislauf-Mitteln, entzündungshemmenden Mitteln, wie z. B. NSAIDs und COX-2-Hemmern, Anthelmintika, Antiarrhythmika, Antibiotika (einschließlich Penicilline), Antikoagulationsmitteln, Antidepressiva, Antidiabetika, Antiepileptika, Antihistaminika, antihypertensiven Mitteln, antimuskarinischen Mitteln, antimykobakteriellen Mitteln, antineoplastischen Mitteln, Immunsuppressiva, antithyroiden Mitteln, antiviralen Mitteln, Anxiolytika, Sedativa (Hypnotika und Neuroleptika), Astringentien, alpha-adrenerger-Rezeptor-Blockern, beta-Adrenoceptor-Blockern, Blutprodukten und -ersatzstoffen, inotropen Herzmitteln, Kontrastmedien, Corticosteroiden, Hustenhemmern (Expektorantien und Mukolytika), diagnostischen Mitteln, diagnostischen Bildgebungsmitteln, Diuretika, Dopaminergika (Antiparkinson-Mittel), Hämostatika, immunologischen Mitteln, lipidregulierenden Mitteln, Muskelrelaxantien, Parasympathomimetika, Parathyroidcalcitonin und Biphosphonaten, Prostaglandinen, Radiopharmaka, Geschlechtshormonen (einschließlich Steroiden), antiallergenen Mitteln, Stimulantien und Anoretika, Sympathomimetika, Thyroidmitteln, Vasodilatoren und Xanthinen.

8. Verfahren gemäß Anspruch 7, wobei die Vorläuferverbindung ausgewählt ist aus der Gruppe bestehend aus Haloperidol, DL-Isoproterenolhydrochlorid, Terfenadin, Propanololhydrochlorid, Desipraminhydrochlorid, Salmeterol, Sildenafilcitrat, Tadalafil, Vardenafil, Fenaminsäuren, Piroxicam, Naproxen, Voltaren (Diclofenac), Rofecoxib, Ibuprofen, Ondansteron, Sumatripan, Naratrypan, Ergotamintartrat plus Coffein, Methylsegid, Olanzapin.

9. Verfahren gemäß einem der vorstehenden Ansprüche, ferner umfassend den Schritt des Entfernens wenigstens eines Teils der festen Trägermatrix, wobei die Nanopartikel eine mittlere Partikelgröße von weniger als 200 nm aufweisen.

10. Verfahren gemäß Anspruch 9, wobei wenigstens 25 % der festen Trägermatrix entfernt werden, bevorzugter wenigstens 50 % der festen Trägermatrix entfernt werden, bevorzugter wenigstens 75 % der festen Trägermatrix entfernt werden, wobei bevorzugter im Wesentlichen die gesamte feste Trägermatrix entfernt wird.

11. Nanopartikelzusammensetzung, umfassend Nanopartikel von Diclofenacsäure in einer festen Trägermatrix dispergiert, wobei die Nanopartikel eine mittlere Größe von weniger als 200 nm aufweisen.

12. Nanopartikelzusammensetzung gemäß Anspruch 11, wobei die mittlere Größe weniger als 100 nm beträgt, die mittlere Größe bevorzugter weniger als 75 nm beträgt, die mittlere Größe bevorzugter weniger als 50 nm beträgt, die mittlere Größe bevorzugter weniger als 30 nm beträgt.

13. Nanopartikelzusammensetzung gemäß Anspruch 11, wobei die feste Trägermatrix wenigstens ein Element ausgewählt aus der Gruppe bestehend aus Na₂CO₃, NaHCO₃, NH₄Cl und NaCl umfasst.

14. Nanopartikelzusammensetzung gemäß Anspruch 13, wobei die feste Trägermatrix entweder Na₂CO₃ allein oder in Kombination mit NaHCO₃ umfasst und die Nanopartikel von Diclofenac in der Form von Diclofenac-Natriumsalz vorliegen.

15. Nanopartikelzusammensetzung gemäß Anspruch 13, wobei die feste Trägermatrix entweder NH₄Cl allein oder in Kombination mit NaCl umfasst und die Nanopartikel von Diclofenac in der Form einer Diclofenacsäure vorliegen.

16. Nanopartikelzusammensetzung, umfassend Nanopartikel von Naproxensäure in einer festen Trägermatrix dispergiert, wobei die feste Trägermatrix entweder NH₄Cl allein oder in Kombination mit NaCl umfasst und die Nanopartikel eine mittlere Größe von weniger als 200 nm aufweisen.

17. Nanopartikelzusammensetzung, umfassend Nanopartikel von Olanzapin in einer festen Trägermatrix dispergiert, wobei die Nanopartikel eine mittlere Größe von weniger als 200 nm aufweisen.

18. Nanopartikelzusammensetzung gemäß Anspruch 17, wobei die mittlere Größe weniger als 100 nm beträgt, die mittlere Größe bevorzugter weniger als 75 nm beträgt, die mittlere Größe bevorzugter weniger als 50 nm beträgt, die mittlere Größe bevorzugter weniger als 30 nm beträgt.

19. Nanopartikelzusammensetzung gemäß Anspruch 17, wobei die feste Trägermatrix NH₄Cl umfasst.

20. Nanopartikelzusammensetzung gemäß Anspruch 17, wobei die Nanopartikel von Olanzapin in der Form vom Olanzapin-HCl vorliegen.

21. Nanopartikelzusammensetzung, umfassend Nanopartikel von Sildenafil in einer festen Trägermatrix dispergiert, wobei die Nanopartikel eine mittlere Größe von weniger als 200 nm aufweisen.

22. Nanopartikelzusammensetzung gemäß Anspruch 21, wobei die mittlere Größe weniger als 100 nm beträgt, die mittlere Größe vorzugsweise weniger als 75 nm beträgt, die mittlere Größe bevorzugter weniger als 50 nm beträgt, die mittlere Größe bevorzugter weniger als 30 nm beträgt.

23. Nanopartikelzusammensetzung gemäß Anspruch 21, wobei die Trägermatrix Citronensäure umfasst.

24. Nanopartikelzusammensetzung gemäß Anspruch 21, wobei die Nanopartikel von Sildenafil in der Form von Sildenafilbase vorliegen.

## Revendications

1. Procédé de production d'une composition de nanoparticules comprenant des nanoparticules d'un agent thérapeutiquement efficace, comprenant l'étape de :
synthèse mécanochimique d'un mélange d'un composé précurseur et d'un co-réactif au moyen d'un milieu de broyage dans un appareil de broyage, pendant une durée suffisante pour produire la composition de nanoparticules comprenant des nanoparticules de l'agent thérapeutiquement efficace dispersé dans une matrice de support solide, où les nanoparticules de l'agent thérapeutiquement efficace sont produites par une réaction chimique à l'état solide entre le composé précurseur et le co-réactif.

2. Procédé de la revendication 1, dans lequel les nanoparticules ont une taille moyenne inférieure à 200 nm, de préférence inférieure à 100 nm, plus préférablement inférieure à 75 nm, plus préférablement inférieure à 50 nm, plus préférablement inférieure à 40 nm.

3. Procédé de l'une quelconque des revendications précédentes, dans lequel la période est comprise entre 5 minutes et 2 heures, plus préférablement la période est comprise entre 5 minutes et 1 heure, plus préférablement la période est comprise entre 5 minutes et 45 minutes, plus préférablement la période est comprise entre 5 minutes et 30 minutes, plus préférablement, la période est comprise entre 10 minutes et 20 minutes.

4. Procédé de l'une quelconque des revendications 1 à 3, dans lequel les milieux de broyage comprennent des billes d'acier.

5. Procédé de la revendication 4, dans lequel les billes d'acier ont un diamètre compris entre 1 et 20 mm, plus préférablement les billes d'acier ont un diamètre compris entre 2 et 15 mm, plus préférablement les billes d'acier ont un diamètre compris entre 3 et 10 mm.

6. Procédé de l'une quelconque des revendications précédentes dans lequel le composé précurseur est choisi dans le groupe constitué d'agents biologiques, acides aminés, protéines, peptides, nucléotides, acides nucléiques et des analogues de ceux-ci.

7. Procédé de l'une quelconque des revendications précédentes, dans laquelle le composé précurseur est choisi dans le groupe constitué de médicaments anti-obésité, stimulants du système nerveux central, caroténoïdes, corticostéroïdes, inhibiteurs d'élastase, antifongiques, thérapies oncologiques, antiémétiques, analgésiques, agents cardiovasculaires, agent anti-inflammatoires, tels que des AINS et des inhibiteurs de COX-2, antihelmintiques, agents antiarythmiques, antibiotiques (comprenant les pénicillines), anticoagulants, antidépresseurs, agents antidiabétiques, antiépileptiques, antihistaminiques, agents antihypertenseurs, agents antimuscariniques, agents antimycobactériens, agents antinéoplasiques, immunosuppresseurs, agents antithyroïdiens, agents antiviraux, anxiolytiques, sédatifs (hypnotiques et neuroleptiques), astringents, agents de blocage des récepteurs alpha-adrénergiques, agents de blocage des bêta-adrénorécepteurs, produits sanguins et succédanés, agents inotropes cardiaques, agents de contraste, corticostéroïdes, antitussifs (expectorants et mucolytiques), agents de diagnostic, agents d'imagerie diagnostique, diurétiques, dopaminergiques (agents antiparkinsoniens), hémostatiques, agents immunologiques, agents de régulation des lipides, des relaxants musculaires, des parasympathomimétiques, la calcitonine parathyroïdienne et des biphosphonates, prostaglandines, agents radiopharmaceutiques, hormones sexuelles (y compris des stéroïdes), agents antiallergiques, stimulants et anorexigènes, sympathomimétiques, agents thyroïdiens, vasodilatateurs et xanthines.

8. Procédé de la revendication 7, dans lequel le composé précurseur est choisi dans le groupe constitué des halopéridol, chlorhydrate de DL-isoprotérénol, terfénadine, chlorhydrate de propranolol, chlorhydrate de désipramine, salmétérol, citrate de sildénafil, tadalafil, vardénafil, acides fénamiques, piroxicam, naproxène, voltarène (diclofénac), rofécoxib, ibuprofène ondanstétron, sumatriptan, naratryptan, ergotamine tartrate plus caféine, méthylségide, olanzapine.

9. Procédé de l'une quelconque des revendications précédentes, comprenant en outre l'étape d'élimination d'au moins une partie de la matrice de support solide, où les nanoparticules ont une taille de particule moyenne inférieure à 200 nm.

10. Procédé de la revendication 9, dans lequel au moins 25 % de la matrice de support solide est éliminée, plus préférablement au moins 50 % de la matrice de support solide est éliminée, plus préférablement au moins 75 % de la matrice de support solide est éliminée, plus préférablement dans lequel sensiblement toute la matrice de support solide est éliminée.

11. Composition de nanoparticules comprenant des nanoparticules de diclofénac acide dispersées dans une matrice de support solide, où les nanoparticules ont une taille moyenne inférieure à 200 nm.

12. Composition de nanoparticules de la revendication 11, dans laquelle la taille moyenne est inférieure à 100 nm, de préférence la taille moyenne est inférieure à 75 nm, plus préférablement la taille moyenne est inférieure à 50 nm, plus préférablement la taille moyenne est inférieure à 30 nm.

13. Composition de nanoparticules de la revendication 11, dans laquelle la matrice de support solide comprend au moins un membre choisi dans le groupe constitué de Na₂CO₃, NaHCO₃, NH₄Cl et NaCl.

14. Composition de nanoparticules de la revendication 13, dans laquelle la matrice de support solide comprend Na₂CO₃ seul ou en combinaison avec NaHCO₃, et les nanoparticules de diclofénac sont sous la forme de sel de sodium de diclofénac.

15. Composition de nanoparticules de la revendication 13, dans laquelle la matrice de support solide comprend NH₄Cl seul ou en combinaison avec NaCl, et les nanoparticules de diclofénac sont sous la forme d'un diclofénac acide.

16. Composition de nanoparticules comprenant des nanoparticules de naproxène acide dispersées dans une matrice de support solide, où la matrice de support solide comprend NH₄Cl seul ou en combinaison avec NaCl, et les nanoparticules ont une taille moyenne inférieure à 200 nm.

17. Composition de nanoparticules comprenant des nanoparticules d'olanzapine dispersées dans une matrice de support solide, dans laquelle les nanoparticules ont une taille moyenne inférieure à 200 nm.

18. Composition de nanoparticules de la revendication 17, dans laquelle la taille moyenne est inférieure à 100 nm, de préférence dans laquelle la taille moyenne est inférieure à 75 nm, plus préférablement dans laquelle la taille moyenne est inférieure à 50 nm, plus préférablement dans laquelle la taille moyenne est inférieure à 30 nm.

19. Composition de nanoparticules de la revendication 17, dans laquelle la matrice de support solide comprend NH₄Cl,

20. Composition de nanoparticules de la revendication 17, dans laquelle les nanoparticules d'olanzapine sont sous la forme d'olanzapine.HCl.

21. Composition de nanoparticules comprenant des nanoparticules de sildénafil dispersées dans une matrice de support solide, dans laquelle les nanoparticules ont une taille moyenne inférieure à 200 nm.

22. Composition de nanoparticules de la revendication 21, dans laquelle la taille moyenne est inférieure à 100 nm, de préférence dans laquelle la taille moyenne est inférieure à 75 nm, plus préférablement dans laquelle la taille moyenne est inférieure à 50 nm, plus préférablement dans laquelle la taille moyenne est inférieure à 30 nm.

23. Composition de nanoparticules de la revendication 21, dans laquelle la matrice de support comprend de l'acide citrique.

24. Composition de nanoparticules de la revendication 21, dans laquelle les nanoparticules de sildénafil sont sous la forme de sildénafil base.
